(19) Europäisches Patentamt
European Patent Office
Office européen des brevets

(11) Publication number: 0 489 220 A1

# (12) EUROPEAN PATENT APPLICATION

(21) Application number: 91100267.3

(22) Date of filing: 10.01.91

(51) Int. Cl.5: A61K 47/48, C07K 5/00, C07K 7/00, A61K 37/02, A61K 39/395, A61K 31/12

(30) Priority: 02.12.90 US 479488

(43) Date of publication of application: 10.06.92 Bulletin 92/24

(84) Designated Contracting States: AT BE CH DE DK ES FR GB GR IT LI NL SE

(71) Applicant: AMERICAN CYANAMID COMPANY
1937 West Main Street P.O. Box 60
Stamford Connecticut 06904-0060(US)

(72) Inventor: Fields, Thomas Lynn
66 Amory Avenue
Pearl River, New York 10965(US)
Inventor: Sassiver, Martin Leon
6 Hadassah Lane
Spring Valley, New York 10977(US)
Inventor: Crockatt, Linda H.
602 Tudor Hill-Old Nyack Turnpike
Spring Valley, New York 10977(US)
Inventor: Upeslacis, Janis
3 Keim Drive
Pomona, New York 10970(US)

(74) Representative: Wächtershäuser, Günter, Dr.
Tal 29
W-8000 München 2(DE)

## (54) Cytotoxic N,N'-bis (succinyl-peptide-) derivatives of 1,4-bis (aminoalkyl)-5,8-dihydroxyanthraquinones and antibody conjugates thereof.

(57) The present invention is concerned with novel N,N'-bis(succinyl-peptide)-derivatives of 1,4-bis(aminoalkyl)-5,8-dihydroxyanthraquinones and the corresponding antibody conjugates thereof each of which have potential use in the treatment of human carcinomas.

EP 0 489 220 A1

## Brief Summary of the Invention

Cytotoxic drug-monoclonal antibody conjugates have potential use in the treatment of human carcinomas as reported by R. W. Baldwin and V. S. Byers, Springer Semin. Immunopathol., 9, 39 (1986); C. W. Pouton, J. Clin Hosp. Pharm., 10, 45 (1985); S. Stuart and R. A. Reisfeld, Eds., "Monoclonal Antibodies in Cancer", Humana Press, Clifton N.J. (1985); C. G. Pitt, et al., J. Controlled Release, 2, 363 (1985); T. Ghose and A. H. Blair, CRC Crit. Rev. Therapeutic Drug Carrier Sys., 3, 262 (1987) and A. T. Freeman and E. Meyhew, Cancer, 58, 573 (1986).

The present invention is concerned with N,N'-bis(succinyl-peptide)derivatives of 1,4-bis(aminoalkyl)-5,8-dihydroxyanthraquinones whose structural formulae Ic or Id appear below and the corresponding antibody conjugates thereof which may be represented by formula If which follows in progression:

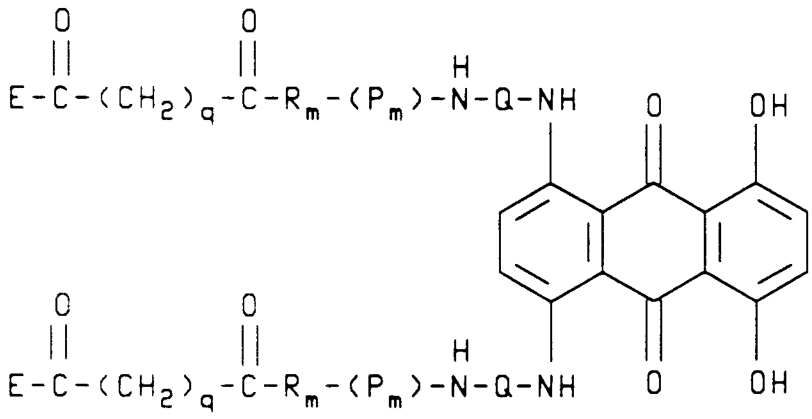

wherein Q is a divalent moiety selected from the group consisting essentially of

$-(CH_2)_n-$, 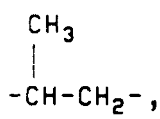, 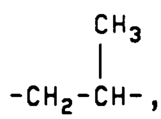, 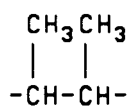,

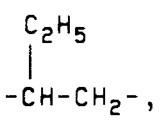, 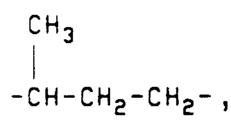, 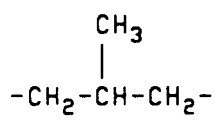

and 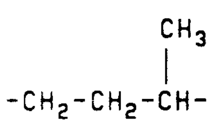

where n is an integer from 2 to 4 inclusive; R is selected from the group of D or L amino acids consisting essentially of cysteine, leucine, isoleucine, phenylalanine, tyrosine, proline, tryptophan, hydroxyproline, aspartic acid, asparagine, glutamic acid, glutamine, lysine, ornithine, arginine, histidine, alanine, glycine, methionine, valine, threonine and serine which are linked through amide bonds between the α-amino functionality of one amino acid and the carboxyl group of the adjacent amino acid, and in those amino acids which possess side-chain functionality, the side-chains can be optionally substituted by amino acid side-chain protecting groups (P), as for example, aspartic acid and glutamic acid can be incorporated containing lower alkyl, benzyl or 4-nitrobenzyl esters as part of the side chain carboxyl group, or lysine and ornithine can contain carbobenzyloxy, tertiary-butyloxy or fluorenylmethoxycarbonyl protecting groups on the side-chain amino functionality, or arginine can be incorporated containing benzyloxycarbonyl, tertiary-butyloxycarbonyl or nitro protection of the guanidinium functionality, or cysteine can be incorporated with tertiarybutyl, tertiary-butylthio or acetyl groups on the side chain sulfhydryl group; E is hydroxyl in formula

Ic or an ester in formula Id; m is an integer from 1 to 10, inclusive; q is an integer from 1 to 4, inclusive; and the pharmacologically-acceptable acid addition salts thereof.

Conjugates of the formula:

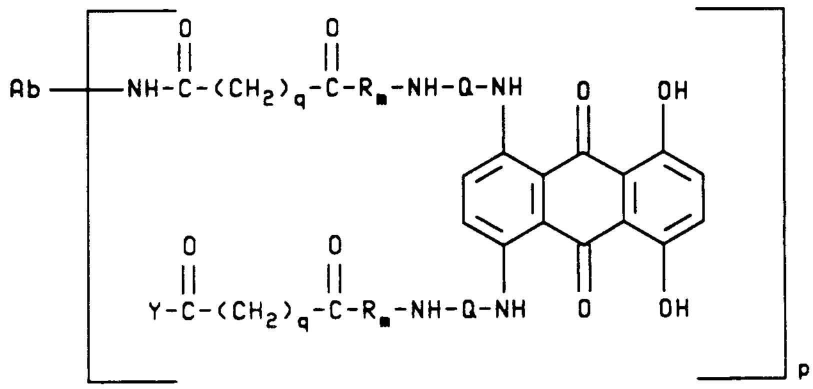

where Q is a divalent moiety selected from the group consisting essentially of

$-(CH_2)_n-$, 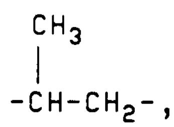 , 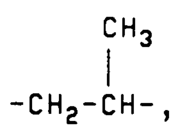 , 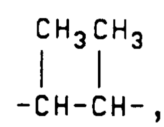 ,

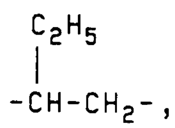 , 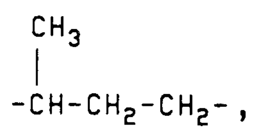 , 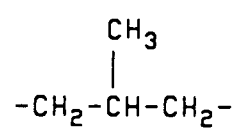

and 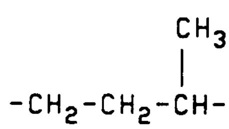

where n is an integer from 2 to 4 inclusive; R is selected from the group of D or L amino acids consisting essentially of cysteine, leucine, isoleucine, phenylalanine, tyrosine, proline, tryptophan, hydroxyproline, aspartic acid, asparagine, glutamic acid, glutamine, lysine, ornithine, arginine, histidine, alanine, glycine, methionine, valine, threonine and serine which are linked through amide bonds between the α-amino functionality of one amino acid, and the carboxyl group of the adjacent amino acid, and in those amino acids which possess side-chain functionality, the side-chains can be optionally substituted by protecting groups (P), as for example, aspartic acid and glutamic acid can be incorporated containing lower alkyl, benzyl or 4-nitrobenzyl esters as part of the side chain carboxyl group, or lysine and ornithine can contain carbobenzyloxy, tertiary-butyloxy or fluorenylmethoxycarbonyl protecting groups on the side-chain amino functionality, or arginine can be incorporated containing benzyloxycarbonyl, tertiary-butyloxycarbonyl or nitro protection of the guanidinium functionality, or cysteine can be incorporated with tertiary-butyl, tertiary-butylthio or acetyl groups on the side chain sulfhydryl group; m is an integer from 1 to 10 inclusive; q is an integer from 1 to 4 inclusive; Ab is a monoclonal or polyclonal antibody or their fragments; Y is hydroxyl or a bond to the antibody, P is the number of molecules of N-hydroxysuccinimide ester reacting per antibody; and the pharmacologically-acceptable acid addition salts thereof.

The synthesis of the N,N'-bis(peptidyl) derivatives, formula Ib, see below, from the compounds of formula Ia, see below, is described in co-pending, commonly-assigned U.S. Patent application, Serial No. 874,195 filed June 13, 1986, which is hereby incorporated by reference:

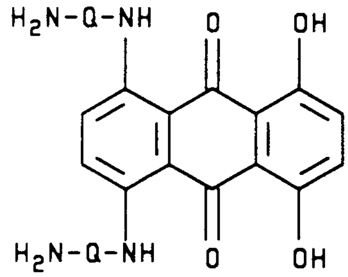

Ia

where Q is as hereinbefore defined and the compounds may be in the form of a pharmacologically acceptable organic or inorganic acid-addition salt or combination of salts; to produce

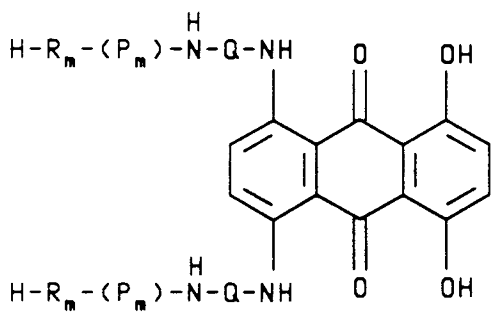

Ib

where Q, R and m are as hereinbefore defined, P is an optionally substituted side-chain protecting or blocking group such as t-butyl ester or the like as hereinbefore described and the compounds may be in the form of a salt as set forth in the description of formula Ia.

The compound of formula Ia where Q is the divalent moiety $-(CH_2)_2-$ is disclosed in U.S. Patents 4,197,249 and 4,526,989. The compounds of formulae Ia and Ib possess the property of inhibiting the growth of leukemia and solid tumors in mammals as determined by the Lymphocytic Leukemia P388 test.

One aspect of this invention comprises modifying the compounds of formula Ib by linking them through a dicarbonyl moiety to an antibody (monoclonal or polyclonal or fragments thereof) directed against antigenic determinants of a given tumor cell type. In this form, targeting to tumor cells occurs by virtue of the antibody specificity, thereby averting at least some of the non-specific toxicity toward normal tissues observed with classical antitumor agents. Attachment of the anticancer agent through a peptide spacer provides a mechanism by which the anticancer agent can be released once targeting to the tumor has occurred. Examples whereby anticancer drugs have been attached to protein carriers such as bovine serum albumin (BSA) have been reported: A. Trouet, et al., U.S. Pat. No. 4,376,765; A. Trouet, et al., p. 19 in "Targeting of Drugs", Plenum Press, New York (1982), as has the evidence that proteolytic enzymes affect the release of drugs subsequent to endocytosis of the complex. However, this concept has not, as yet, been applied to delivery of anticancer drugs by antibodies since those peptide sequences generally used to attach drugs to protein such as BSA, because of their hydrophobic nature, have not been useful for conjugating the same drugs to antibodies. Antibodies function in part by precipitating antigen from circulation, so it would be expected that conjugation of drugs to antibodies using hydrophobic spacers would lead to precipitation of antibody, or at best, generate complexes which contain very low levels of drugs. These limitations have been overcome through one aspect of the invention by incorporating glutamic or aspartic acid as members of the peptide sequence. Since amino acids are ionized at physiological pH,

they help to solubilize the drug-antibody conjugates and thus permit conjugation of substantially more drugs per antibody.

In greater detail, compounds of the type represented by formula Ib are reacted with a $C_1$ to $C_4$ dicarboxylic anhydride, for example succinic anhydride, in the presence of a tertiary amine base such as triethylamine to provide compounds of the formula Ic (which may be in the salt form):

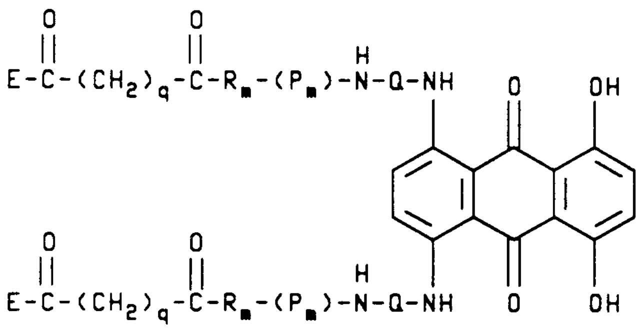

Ic or Id

where Q, R, P, q and m are as hereinbefore defined, and E is hydroxyl, and whose terminal carboxyl groups are activated for reaction with antibody amino groups by esterification with N-hydroxy-succinimide/dicyclohexylcarbodiimide to furnish the compounds or salts of the formula Id, where Q, R, P and m are as hereinbefore defined and E is

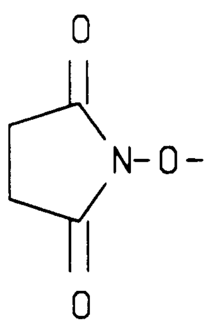

In a preferred embodiment of this invention, q is 2.

The compounds of formulae Ic and Id have been shown to possess the property of inhibiting the growth of leukemia in mammals as determined by the Lymphocytic Leukemia P-388 test. Results of this test on representative compounds of formulae Ic and Id appear in Table I hereinbelow.

Prior to reaction with an antibody or its fragments the side chain protecting groups ($P_m$) as hereinabove described are removed by mild acid treatment of the compounds of formula Id to generate the compounds of formula Ie (which may be in the salt form):

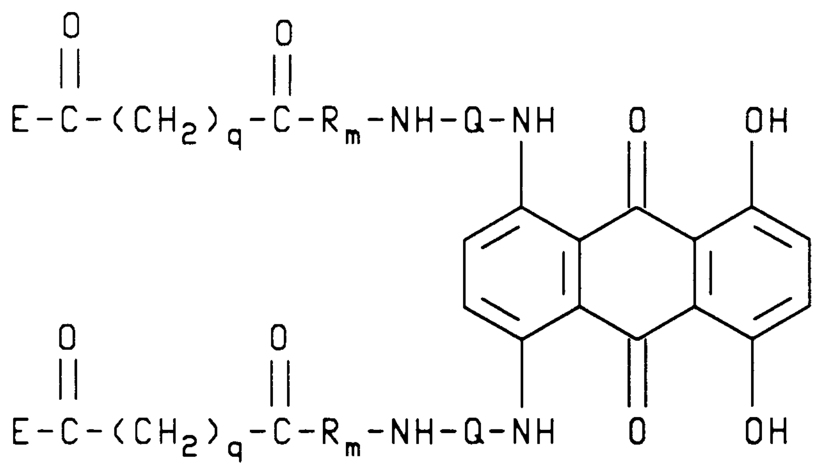

Ie

wherein Q, R, m, q, and E are as hereinabove defined.

Compounds of formulae Ie are coupled with antibodies to generate conjugates of formulae If. Anticancer testing results on representative members of these conjugates can be found in Table II hereinbelow.

## DETAILED DESCRIPTION OF THE INVENTION

The compounds of this invention may be prepared according to the following reaction sequences, wherein the compounds may also be in the form of pharmacologically-acceptable acid addition salts:

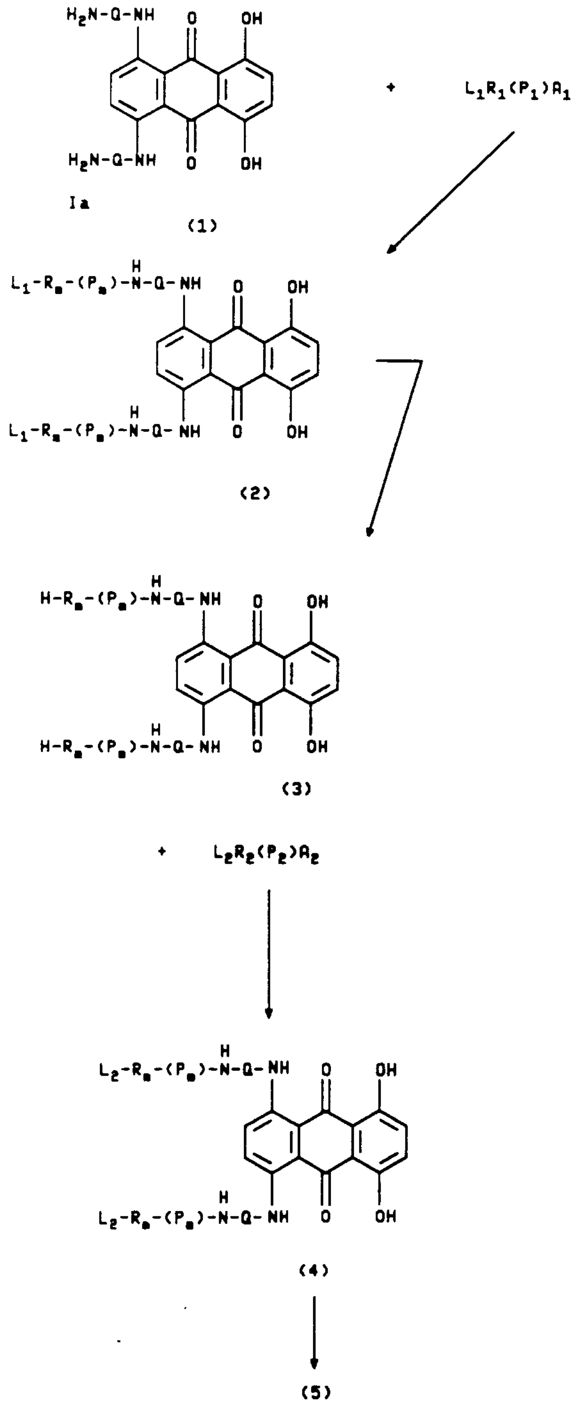


(1) (2) (3) (4) (5)

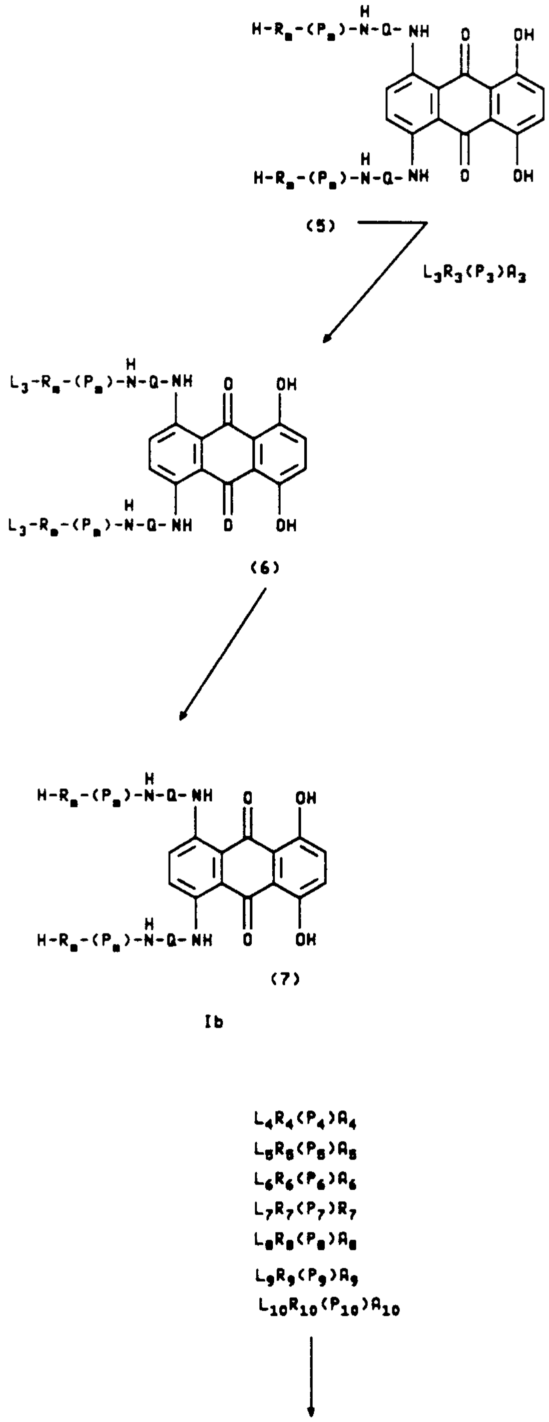
$L_3R_3(P_3)A_3$
$L_4R_4(P_4)A_4$
$L_5R_5(P_5)A_5$
$L_6R_6(P_6)A_6$
$L_7R_7(P_7)R_7$
$L_8R_8(P_8)A_8$
$L_9R_9(P_9)A_9$
$L_{10}R_{10}(P_{10})A_{10}$

(5)

(6)

(7)

Ib

(8)

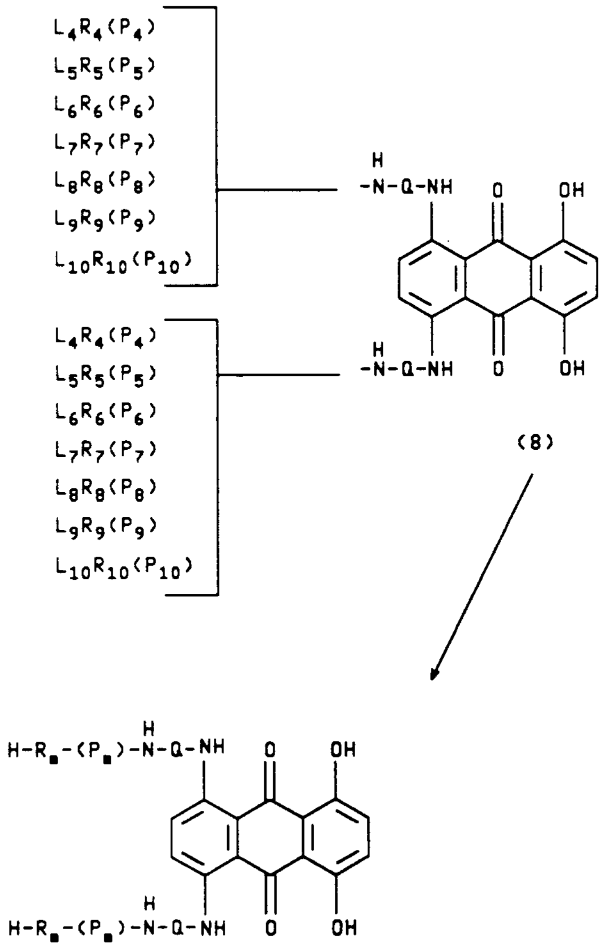

(8)

(9)

Ib

(9) +

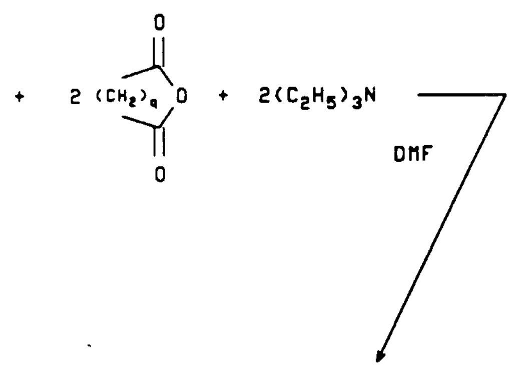


(10)

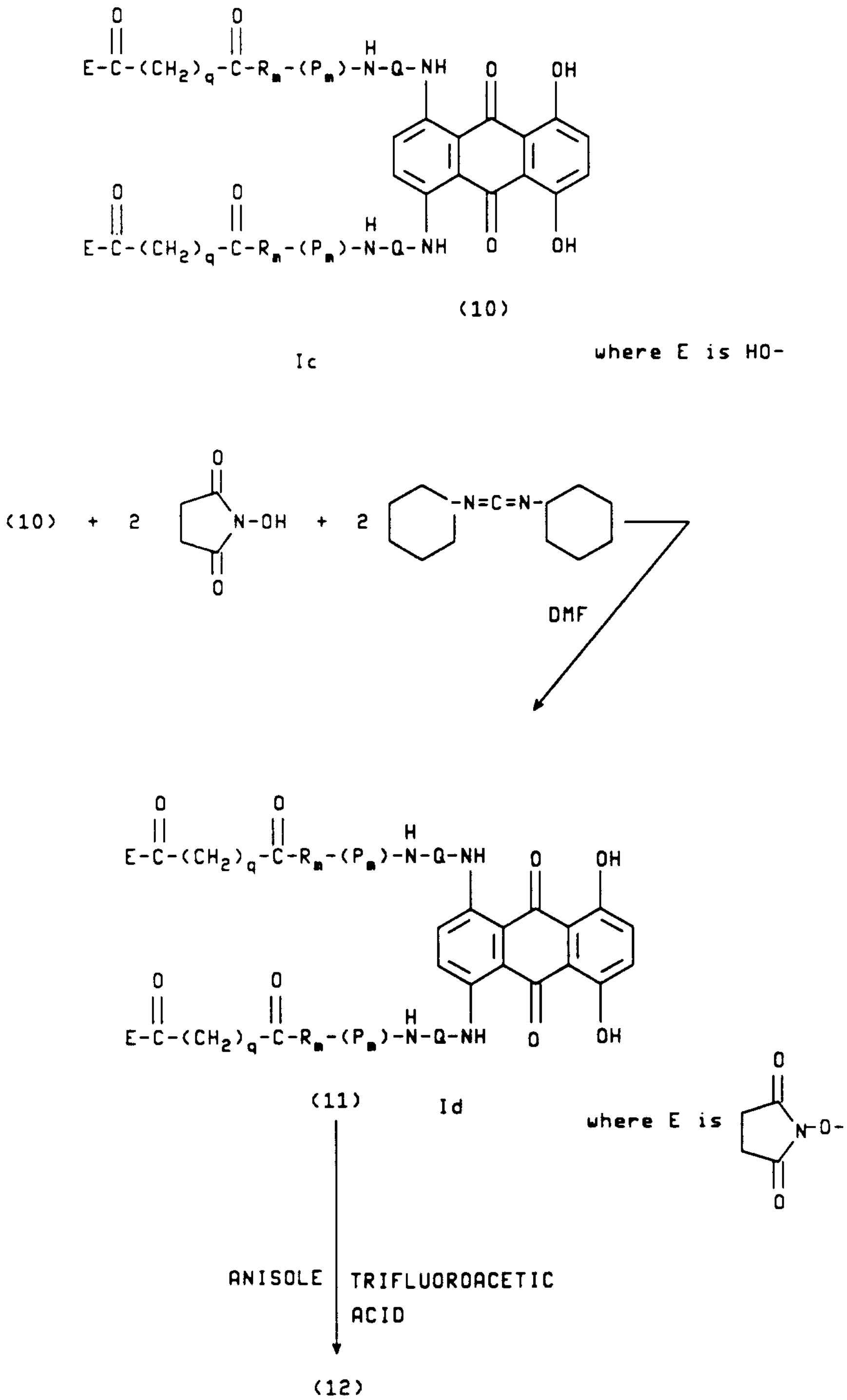


(10)

Ic

where E is HO-

(10) + 2 + 2

(11)

Id

where E is

(12)

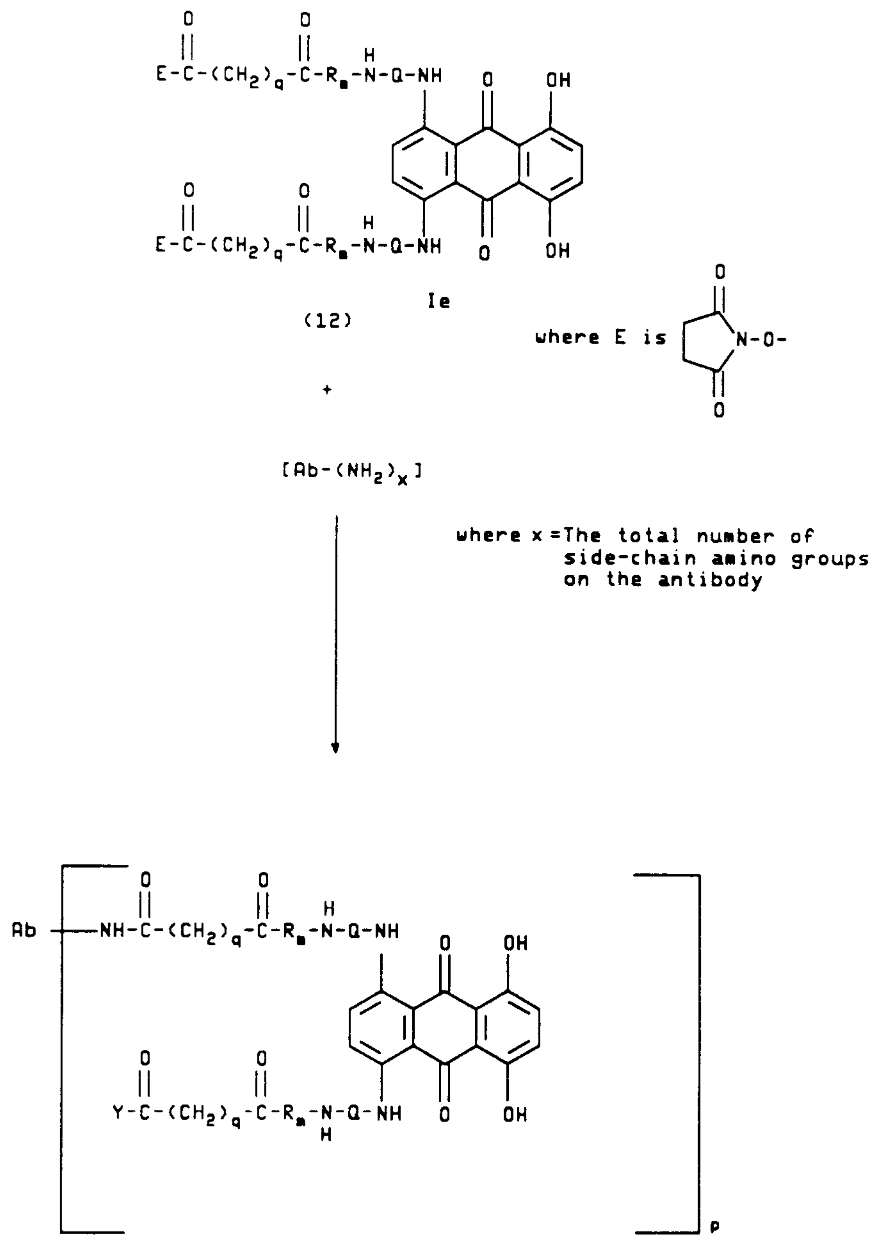


(13) If

In accordance with the above reaction scheme, the anthraquinone (1), formula Ia, is dissolved in a solvent such as N,N-dimethylformamide containing an organic base such as triethylamine or diisopropylethylamine, or alternatively, in dry tetrahydrofuran in the presence of trimethylsilyl chloride and triethylamine. The solution is chilled, and to this is added the first amino acid residue $R_1$ activated $A_1$ through its carboxyl group as its corresponding hydroxysuccinimide ester, or as the isobutyryl chloroformate, or as a mixed or symmetrical anhydride, or as any one of a number of carboxyl activating functionalities known to those skilled in the art of peptide synthesis. The $\alpha$-amino group of the activated acid must be protected $L_1$ at this point by a group such as tertiary-butyloxycarbonyl, benzyloxycarbonyl, fluorenylmethoxycarbonyl, and the like, to avoid polymerization during condensation.

Similarly, those amino acids which contain functionality in their side-chains in general also need to have the functionality protected ($P_1$), and are selected as described previously. The protecting groups used on the side chain can be the same or different than those used to protect the $\alpha$-amino radical although differential protection is preferred if additional amino acid groups are to be added. The activated, protected amino acid [$L_m$-$R_m$-($P_m$)-$A_m$], e.g. [$L_1$-$R_1$-($P_1$)-$A_1$] where $m = 1$, is dissolved in the same solvent used to dissolve the anthraquinone, and addition is done dropwise with stirring The reaction is stirred at O° to 40°C,

preferably at room temperature, for about 24 hours, then filtered and the desired amide (2) is isolated either by precipitation with a solvent of low polarity, or by evaporation.

The protecting group $L_1$ on the α-amine is then removed such that elongation of the peptide chain can be achieved if desired. For example, the tertiary-butyloxycarbonyl group can be removed by dissolving (2) in anisole, cooling the solution in an ice bath and adding trifluoroacetic acid. The solution is stirred briefly in the cold, then warmed to room temperature for 1-24 hours. The desired deprotected product (3) is isolated by diluting the reaction with a solvent in which the product is not soluble, for example, diethyl ether, and the precipitate collected by filtration.

Alternatively, the tertiary-butyloxycarbonyl group can be removed by dissolving the compound (2) in a mixture of acetic acid and anisole, then bubbling hydrogen chloride gas into the solution for a few minutes. After standing at room temperature for 1-24 hours, the absence of (2) is determined by a technique such as thin layer chromatography or analytical high pressure liquid chromatography, then the product (3) is isolated by precipitation as described above. A benzyloxycarbonyl group can be removed by dissolving (2) in a solvent such as acetic acid, cooling, but not freezing the solution, and bubbling in gaseous hydrobromic acid. After the reaction has remained at room temperature for 1-24 hours and the absence of starting material has been determined, the product (3) is isolated by precipitation.

Alternatively, the benzyloxycarbonyl group can be removed by hydrogenation of (2) in the presence of a noble metal catalyst such as palladium on carbon, but in this case re-oxidation of the reduced anthraquinone ring is generally necessary. A fluorenylmethyloxycarbonyl group can be removed by dissolving (2) in a polar solvent such as N,N-dimethylformamide and adding a secondary amine such as dimethylamine. The product (3) is again isolated either by precipitation or by evaporation of the reaction solution.

If desired, the next amino acid fragment ($R_m$ where m is 2), is then added by repeating the sequence of reacting (3) with an α-amino and side-chain protected, carboxy group activated amino acid derivative [$L_2$-$R_2$-($P_2$)-$A_2$], isolating the intermediate (4) and removing the α-amino protecting group as described above to obtain the product (5). The process is repeated by judicious manipulation of the above conditions, or by applying conditions familiar to those skilled in the art of peptide synthesis until the entire desired peptide sequence (9) formula Ib has been assembled.

Alternatively, the entire peptide sequence can be assembled prior to formation of the amide bond between the peptide carboxy terminus and the anthraquinone nucleus. This can be accomplished by applying the solution techniques described above or assembling the peptide chain using any one of the techniques which have been developed as modifications of the Merrifield solid phase peptide synthesis procedure.

The compound of the entire peptide sequence of formula Ib (9), containing all side-chain protecting groups ($P_m$), is dissolved in a solvent such as N,N-dimethylformamide and reacted with a $C_1$ to $C_4$ dicarboxylic anhydride (q = 1-4) such as succinic anhydride in the presence of a tertiary amine base such as triethylamine (shown in the reaction sequence), diisopropylethylamine or N-methylmorpholine by stirring at room temperature for about 8-48 hours. The product is isolated by diluting the reaction mixture with a solvent in which the product is not soluble in, for example, diethyl ether. The precipitate is collected by filtration, dried in vacuo for several hours, then triturated with cold water and acidified to pH 2.0 with dilute phosphoric acid. Then the product (10) formula Ic where E is hydroxyl, is collected, washed with cold water and dried.

A solution of about 1 equivalents of the product (10) in N,N-dimethylformamide in the presence of about 2.2 equivalents of N-hydroxysuccinimide and 2.2 equivalents of dicyclohexylcarbodiimide is stirred for from 16-74 hours, if necessary making several more additions of 1.1 equivalents in portions of N-hydroxysuccinimide and dicyclohexylcarbodiimide until the absence of starting material (10) is determined by thin layer chromatography. The mixture is filtered with dry equipment and the filtrate is evaporated to dryness. The residue is triturated with a solvent such as ethyl acetate, then filtered, dried and purified by stirring in a solvent such as isopropyl alcohol to give the hydroxysuccinimide ester product (11), formula Id.

Removal of side-chain protecting groups, where desirable, and where these groups are the tertiary-butyloxycarbonyl, benzyloxycarbonyl or fluorenylmethyloxycarbonyl radicals is accomplished as described above. In addition, the tertiary-butyloxy group required for protection of aspartic and glutamic acids is removed under the acid hydrolysis conditions described for cleavage of the tertiary-butyloxycarbonyl protecting group. Most other groups are removed by slight modifications of the above-described procedures to give the product (12), formula Ie, where E is an ester.

More specifically it was found most desirable to include an aspartic or glutamic acid moiety in the peptide linker portion of the molecules of formulae Ia-Id to provide sufficient water solubility in the pH 6-9 range to these reagents and the antibody conjugates made from them. Without this provision, reagent

and/or conjugate precipitation resulted in low yields of the desired conjugates.

Coupling of the succinimidoyl esters (12) with antibodies (Ab) or their fragments, containing reactive amino groups (Ab-$(NH_2)_x$] such as for example, the ϵ-amino groups of a lysine moiety, and wherein x designates the total number of such primary amino groups of an antibody (commonly around 50 to 100), in aqueous buffers at a pH range of 6-9 at 0°C to 40°C, inclusive, for 1-24 hours, inclusive, as shown in the reaction scheme to give the antibody conjugate (13) If, where Ab is antibody- or its fragments, Q, R, and m are as hereinbefore described, Y is -OH or a bond to the antibody and p is the number of Ie molecules reacting per antibody.

Antibodies which may be used in accordance with this invention include monoclonal antibodies and polyclonal antibodies. A number of different monoclonal antibodies (MoAbs) such as MoAbs Lym 1 and Lym 2 may be used. The production and characteristics of these MoAbs (which recognize different antigens on mature B-lymphocytes and their product lymphomas) are described by A. L. Epstein, et al., Cancer Research, 47, 830 (1987). MoAb B72.3 targets primarily to carcinomas of the breast and colon, though reactivity with pancreatic, ovarian, and lung carcinomas has also been noted. The antibody has been described by T. L. Klug, et al., Int. J. Cancer, 38, 661 (1986). MoAb CT-M-01, which recognizes primarily breast tumors is described in EPO application 86 401482.4 filed July 3, 1987 and MAC-68 is produced by a sub-clone of the hybridoma which produces CT-M-01, and recognizes both breast and colon carcinomas.

The monoclonal antibody designated p96.5 recognizes a 97 kilodalton antigen on human melanomas, as described by J.P. Brown, et al., Proc. Nat'l Acad. Sci., 78, 539 (1981). The T-101 antibody, described by I. Royston, et al., J. Immunol., 125, 725 (1980) targets an antigen on adult T-cell leukemias, and the antibodies designated MX-1/16.4, MX-1/57.1.4, and MX-1/6.2 target the MX-1 human mammary tumor line.

It should not, however, be construed that this invention is limited to or restricted by the aforementioned antibodies. Instead, the methodology is sufficiently general that it can be applied to all antibodies regardless of their class or isotype, their enzymatically-derived fragments, their chemically manipulated and stabilized fragments, as well as their respective chimeric and humanized counterparts. Nor are the targeting units restricted only to monoclonal antibodies; polyclonal antibodies are also within the scope of the invention.

The conjugates If were purified by gel filtration and dialysis. Their chemical and biological properties were evaluated in a series of tests briefly outlined below.

| Test or Measurement | Property Evaluated |
|---|---|
| 1) High performance size exclusion liquid chromatography | general purity; extent of aggregation |
| 2) Ultraviolet absorption spectroscopy | molar incorporation of reagent into the antibody. |
| 3) Radioimmune (or Elisa) assay | effect of conjugation on antibody binding |
| 4) Treatment of experimental tumor in nude mice | cytotoxicity targeting of conjugates. |

Certain of the novel intermediate N,N'-bis(succinimidoyl peptide)derivatives of 1,4-bis(amino alkyl)-5,8-dihydroxyanthraquinones, prior to conjugation to antibodies, possess the property of inhibiting the growth of leukemia in mammals as established by the following test.

## Lymphocytic Leukemia P388 Test

The animals used were BDFI mice, all of one sex, weighing a minimum of 18 g and all within a 3 g weight range. There were 5 or 6 mice per test group. The tumor transplant was by intraperitoneal injection of 0.5 ml of dilute ascitic fluid containing $10^6$ cells of lymphocytic leukemia P388. The test compounds were administered intraperitoneally on days 1, 5 and 9 (relative to tumor inoculation) at various doses. The animals were weighed and survivors recorded on a regular basis for 60 days. The median survival time and the ratio of survival time for treated (T)/control (C) animals were calculated The positive control compound was 5-fluorouracil. The control was saline. The results of this test appear in Table I.

TABLE I

Lymphocytic Leukemia P388

| Compound | Dose (mg/kg) | Median Survival (Days) | T/Cx100 (%) |
|---|---|---|---|
| N,N'-[(9,10-Dihydro-5,8-dihydroxy-9,10-dioxo-1,4-anthracenediyl)bis-(imino-2,1-ethanediyl)]bis-N-(3-carboxy-1-oxopropyl-L-leucyl-L-alanyl-L-leucinamide] | 3.0 | 20.5 | 186 |
| | 1.5 | 20.0 | 182 |
| | 0.75 | 19.5 | 177 |
| Control | - | 11.0 | - |
| 5-Fluorouracil | 60 | 21.0 | 191 |
| N,N'-[(9,10-Dihydro-5,8-dihydroxy-9,10-dioxo-1,4-anthracenediyl)bis(imino-2,1-ethanediyl)]bis[N-(3-carboxy-1-oxopropyl)-glycylglycyl-L-leucinamide] | 3.0 | 12.0 | 120 |
| | 1.5 | 10.5 | 105 |
| | 0.75 | 11.0 | 110 |
| Control | - | 10.0 | - |
| 5-Fluorouracil | 60 | 22.0 | 220 |

TABLE I (continued)

| Compound | Dose (mg/kg) | Median Survival (Days) | T/Cx100 (%) |
|---|---|---|---|
| N,N'-[(9,10-Dihydro-5,8-dihydroxy-9,10-dioxo-1,4-anthracenediyl)bis(imino-2,1-ethanediyl)]bis[N-(3-carboxy-1-oxopropyl)-L-alanyl-L-alanyl-L-alaninamide] | 3.0<br>1.5<br>0.75 | 18.5<br>18.0<br>16.5 | 185<br>180<br>165 |
| Control | - | 10.0 | - |
| 5-Fluorouracil | 60 | 22.0 | 220 |
| N,N'-[(9,10-Dihydro-5,8-dihydroxy-9,10-dioxo-1,4-anthracenediyl)bis(imino-2,1-ethanediyl)]bis[N-(3-carboxy-1-oxopropyl)-L-alanyl-L-alanyl-L-alaninamide] | 3.0<br>1.5<br>0.75 | 15.0<br>14.0<br>12.0 | 143<br>133<br>114 |
| Control | - | 10.5 | - |
| 5-Fluorouracil | 60 | 20.5 | 195 |

TABLE I (continued)

| Compound | Dose (mg/kg) | Median Survival (Days) | T/Cx100 (%) |
|---|---|---|---|
| N,N'-[(9,10-Dihydro-5,8-dihydroxy-9,10-dioxo-1,4-anthrancenediyl)-bis(imino-2,1-ethanediyl)]bis[(3-carboxy-1-oxopropyl)-L-alanyl-L-leucinamide] | 3.0 | 16.0 | 160 |
| | 1.5 | 12.0 | 120 |
| | 0.75 | 11.0 | 110 |
| Control | - | 10.0 | - |
| 5-Fluorouracil | 60 | 21.0 | 210 |
| N,N'-[(9,10-Dihydro-5,8-dihydroxy-9,10-dioxo-1,4-anthracenediyl)bis(imino-2,1-ethanediyl)]bis[(3-carboxy-1-oxopropyl)-L-alanyl-L-alaninamide] | 3.0 | 11.0 | 110 |
| | 1.5 | 10.5 | 105 |
| | 0.75 | 11.0 | 110 |
| Control | - | 10.0 | - |
| 5-Fluorouracil | 60 | 21.0 | 210 |

TABLE I

Lymphocytic Leukemia P388

| Compound | Dose (mg/kg) | Median Survival (Days) | T/Cx100 (%) |
|---|---|---|---|
| N, N'-[(9,10-Dihydro-5,8-dihydroxy-9,10-dioxo-1,4-anthracenediyl)bis(imino-2,1-ethanediyl)]bis[N-(3-carboxy-1-oxopropyl)-L-α-aspartyl-L-seryl-L-alaninamide] bis(1,1-dimethylethyl)ester | 3.0<br>1.5<br>0.75 | 19.0<br>14.5<br>13.0 | 173<br>132<br>118 |
| Control | - | 11.0 | - |
| 5-Fluorouracil | 60 | 21.0 | 191 |
| N,N'-[(9,10-Dihydro-5,8-dihydroxy-9,10-dioxo-1,4-anthracenediyl)bis(imino-2,1-ethanediyl)]bis[N-[4-[(2,5-dioxo-1-pyrrolidinyl)oxy]-1,4-dioxobutyl]-L-α-aspartyl-L-seryl-L-alanamide] bis(1,1-dimethylethyl)ester | 3.0<br>1.5<br>0.75 | 24.5<br>20.0<br>18.0 | 204<br>167<br>150 |
| Control | - | 11.0 | - |
| 5-Fluorouracil | 60 | 21.5 | 179 |

TABLE I (continued)

| Compound | Dose (mg/kg) | Median Survival (Days) | T/Cx100 (%) |
|---|---|---|---|
| N,N′-[(9,10-Dihydro-5,8-dihydroxy-9,10-dioxo-1,4-anthracenediyl)bis(imino-2,1-ethanediyl)]-bis[N-(3-carboxy-1-oxopropyl-L-α-aspartyl-L-alanyl-L-alaninamide]bis(1,1-dimethylethyl)ester | 3.0<br>1.5<br>0.75 | 18.5<br>16.0<br>15.0 | 168<br>145<br>136 |
| Control | - | 11.0 | - |
| 5-Fluorouracil | 60 | 23.0 | 209 |
| N,N′-[(9,10-Dihydro-5,8-dihydroxy-9,10-dioxo-1,4-anthracenediyl)bis(imino-2,1-ethanediyl)]bis[N-(3-carboxy-1-oxopropyl-L-α-aspartyl-L-alanyl-L-leucinamide] bis(1,1-dimethylethyl)ester | 3.0<br>1.5<br>0.75 | 22.0<br>19.5<br>19.0 | 200<br>177<br>173 |
| Control | - | 11.0 | - |
| 5-Fluorouracil | 60 | 22.0 | 200 |

TABLE I (continued)

| Compound | Dose (mg/kg) | Median Survival (Days) | T/Cx100 (%) |
|---|---|---|---|
| N,N'-[(9,10-Dihydro-5,8-dihydroxy-9,10-dioxo-1,4-anthracenediyl)-bis(imino-2,1-ethanediyl)]bis[N-(3-carboxy-1-oxopropyl)-L-α-aspartyl-L-alanyl-L-alaninamide] | 3.0<br>1.5 | 14.5<br>12.0 | 132<br>109 |
| Control | - | 11.0 | - |
| 5-Fluorouracil | 60 | 11.5 | 105 |
| N,N'-[(9,10-Dihydro-5,8-dihydroxy-9,10-dioxo-1,4-anthracenediyl)bis(imino-2,1-ethanediyl)]bis[N-[4-[(2,5-dioxo-1-pyrrolidinyl)oxy]-1,4-dioxobutyl]-L-α-aspartyl-L-alanyl-L-alaninamide] bis(1,1-dimethylethyl)ester | 3.0<br>1.5<br>0.75 | 29.5<br>22.5<br>21.0 | 268<br>205<br>191 |
| Control | - | 11.0 | - |
| 5-Fluorouracil | 60 | 11.5 | 105 |

TABLE I (continued)

| Compound | Dose (mg/kg) | Median Survival (Days) | T/Cx100 (%) |
|---|---|---|---|
| N,N'-[(9,10-Dihydro-5,8-dihydroxy-9,10-dioxo-1,4-anthracenediyl)bis(imino-2,1-ethanediyl)]bis[N-(3-carboxy-1-oxopropyl)-L-α-aspartyl-L-alanyl-L-leucinamide] | 3.0<br>1.5<br>0.75 | 19.0<br>14.0<br>12.0 | 190<br>140<br>120 |
| Control | - | 10.0 | - |
| 5-Fluorouracil | 60 | 22.0 | 220 |
| N,N'-[(9,10-Dihydro-5,8-dihydroxy-9,10-dioxo-1,4-anthracenediyl)bis(imino-2,1-ethanediyl)]bis[N-[4-[(2,5-dioxo-1-pyrrolidinyl)oxy]-1,4-dioxobutyl]-L-α-aspartyl-L-alanyl-L-alaninamide] | 3.0<br>1.5<br>0.75 | 14.0<br>12.0<br>11.0 | 140<br>120<br>110 |
| Control | - | 10.0 | - |
| 5-Fluorouracil | 60 | 22.0 | 220 |

TABLE I (continued)

| Compound | Dose (mg/kg) | Median Survival (Days) | T/Cx100 (%) |
|---|---|---|---|
| N,N'-[(9,10-Dihydro-5,8-dihydroxy-9,10-dioxo-1,4-anthracenediyl)bis(imino-2,1-ethanediyl)]bis[N-(3-carboxy-1-oxopropyl)-L-α-aspartyl-L-seryl-L-alaninamide] | 3.0<br>1.5<br>0.75 | 19.0<br>16.0<br>14.0 | 158<br>133<br>117 |
| Control | - | 12.0 | - |
| 5-Fluorouracil | 60 | 21.5 | 179 |
| N,N'-[(9,10-Dihydro-5,8-dihydroxy-9,10-dioxo-1,4-anthracenediyl)bis(imino-2,1-ethanediyl)bis[N-[4-[(2,5-dioxo-1-pyrrolidinyl)oxy]-1,4-dioxobutyl]-L-α-aspartyl-L-alanyl-L-leucinamide] bis(1,1-dimethylethyl)ester | 3.0<br>1.5<br>0.75 | 22.0<br>23.0<br>19.5 | 200<br>209<br>177 |
| Control | - | 11.0 | - |
| 5-Fluorouracil | 60 | 20.0 | 182 |

TABLE I (continued)

| Compound | Dose (mg/kg) | Median Survival (Days) | T/Cx100 (%) |
|---|---|---|---|
| N,N'-[(9,10-Dihydro-5,8-dihydroxy-9,10-dioxo-1,4-anthracenediyl)bis(imino-2,1-ethanediyl)]bis[N-[4-[(2,5-dioxo-1-pyrrolidinyl)oxy]-1,4-dioxobutyl]-L-α-aspartyl-L-alanyl-L-leucinamide] | 3.0<br>1.5<br>0.75 | 20.0<br>16.0<br>12.5 | 182<br>145<br>114 |
| Control | - | 11.0 | - |
| 5-Fluorouracil | 60 | 20.0 | 182 |
| N,N'-[(9,10-Dihydro-5,8-dihydroxy-9,10-dioxo-1,4-anthracenediyl)bis(imino-2,1-ethanediyl)]bis[N-(3-carboxy-1-oxopropyl)-L-arginyl-L-seryl-L-alaninamide], dihydrobromide | 3.0<br>1.5<br>0.75 | 22.5<br>20.0<br>18.5 | 205<br>182<br>168 |
| Control | - | 11.0 | - |
| 5-Fluorouracil | 60 | 20.0 | 182 |

TABLE I (continued)

| Compound | Dose (mg/kg) | Median Survival (Days) | T/Cx100 (%) |
| --- | --- | --- | --- |
| N,N'-[(9,10-Dihydro-5,8-dihydroxy-9,10-dioxo-1,4-anthracenediyl)bis(imino-2,1-ethanediyl)]bis[N-(3-carboxy-1-oxopropyl)glycylglycinamide] | 3.0<br>1.5<br>0.75 | 14.0<br>12.0<br>12.0 | 140<br>120<br>120 |
| Control | - | 10.0 | - |
| 5-Fluorouracil | 60 | 21.0 | 210 |
| N,N'-[(9,10-Dihydro-5,8-dihydroxy-9,10-dioxo-1,4-anthracenediyl)bis(imino-2,1-ethanediyl)]bis[N-(3-carboxy-1-oxopropyl)L-alanyl-L-alanyl-L-argininamide], dihydrobromide | 3.0<br>1.5<br>0.75 | 17.0<br>14.5<br>13.5 | 170<br>145<br>135 |
| Control | - | 10.0 | - |
| 5-Fluorouracil | 60 | 21.0 | 210 |

TABLE I (continued)

| Compound | Dose (mg/kg) | Median Survival (Days) | T/Cx100 (%) |
|---|---|---|---|
| N,N'-[(9,10-Dihydro-5,8-dihydroxy-9,10-dioxo-1,4-anthracenediyl)bis(imino-2,1-ethanediyl)]bis[N-(3-carboxy-1-oxopropyl)L-leucyl-L-alaninamide] | 3.0<br>1.5<br>0.75 | 17.5<br>15.0<br>12.0 | 175<br>150<br>120 |
| Control | - | 10.0 | - |
| 5-Fluorouracil | 60 | 20.0 | 200 |
| N,N'-[(9,10-Dihydro-5,8-dihydroxy-9,10-dioxo-1,4-anthracenediyl)bis(imino-2,1-ethanediyl)]bis[N-(3-carboxy-1-oxopropyl)glycyl-L-α-aspartyl-L-leucyl-L-alanyl-L-leucinamide] bis(1,1-dimethylethyl) ester | 3.0<br>1.5<br>0.75 | 20.5<br>20.0<br>18.0 | 205<br>200<br>180 |
| Control | - | 10.0 | - |
| 5-Fluorouracil | 60 | 22.0 | 220 |

TABLE I (continued)

| Compound | Dose (mg/kg) | Median Survival (Days) | T/Cx100 (%) |
|---|---|---|---|
| N,N'-[(9,10-Dihydro-5,8-dihydroxy-9,10-dioxo-1,4-anthracenediyl)bis(imino-2,1-ethanediyl)]bis[N-(3-carboxy-1-oxopropyl)-L-α-aspartyl-L-leucyl-L-alanyl-L-leucinamide] | 3.0<br>1.5<br>0.75 | 19.0<br>19.0<br>18.0 | 190<br>180<br>180 |
| Control | - | 10.0 | - |
| 5-Fluorouracil | 60 | 22.0 | 220 |
| N,N'-[(9,10-Dihydro-5,8-dihydroxy-9,10-dioxo-1,4-anthracenediyl)bis[imino-2,1-ethanediylimino[1-[2-(1,1-dimethylethoxy)2-oxoethyl]-2-oxo-2,1-ethanediyl]]]bis[N-(3-carboxy-1-oxopropyl)-L-leucyl-L-alanyl-L-leucinamide] | 3.0<br>1.5<br>0.75 | 20.5<br>19.5<br>19.0 | 205<br>195<br>190 |
| Control | - | 10.0 | - |
| 5-Fluorouracil | 60 | 21.0 | 210 |

TABLE I (continued)

| Compound | Dose (mg/kg) | Median Survival (Days) | T/Cx100 (%) |
|---|---|---|---|
| N,N'-[(9,10-Dihydro-5,8-dihydroxy-9,10-dioxo-1,4-anthracenediyl)bis(imino-2,1-ethanediyl)]bis[N-(3-carboxy-1-oxopropyl)-L-α-aspartyl-L-seryl-L-alanyl-L-leucyl-L-leucinamide] | 3.0<br>1.5<br>0.75 | 22.0<br>19.5<br>17.0 | 220<br>195<br>170 |
| Control | - | 10.0 | - |
| 5-Fluorouracil | 200 | 16.5 | 165 |
| N,N'-[(9,10-Dihydro-5,8-dihydroxy-9,10-dioxo-1,4-anthracenediyl)bis(imino-2,1-ethanediyl)]bis[N-(3-carboxy-1-oxopropyl)glycylglycyl-L-α-aspartyl-L-alaninamide] | 3.0<br>1.5 | 14.0<br>11.0 | 140<br>110 |
| Control | - | 10.0 | - |
| 5-Fluorouracil | 200 | 16.5 | 165 |

TABLE I (continued)

| Compound | Dose (mg/kg) | Median Survival (Days) | T/Cx100 (%) |
|---|---|---|---|
| N,N'-[(9,10-Dihydro-5,8-dihydroxy-9,10-dioxo-1,4-anthracenediyl)bis(imino-2,1-ethanediyl)]bis[N-(3-carboxy-1-oxopropyl)glycyl-L-phenylalanyl-L-phenylalanyl-L-leucinamide] | 3.0 | 18.0 | 180 |
| | 1.5 | 18.5 | 185 |
| | 0.75 | 18.0 | 180 |
| Control | - | 10.0 | - |
| 5-Fluorouracil | 60 | 22.0 | 220 |

Certain of the novel conjugates Ab-N,N'-bis(succinimidoylpeptide) derivatives of 1,4-bis(aminoalkyl)-5,8-dihydroxyanthraquinones possess the property of inhibiting the growth of target tumors in mammals as established by the following test.

Inhibition of tumor growth by antitumor antibody conjugates

Several conjugates derived from monoclonal antibody p96.5 (J. P. Brown et al., Proc. Natl. Acad. Sci., 78, 539-543 (1981)) were tested for inhibition of its target human melanoma cell line SK-Mel-28 in athymic mice. Female Balb/nude (athymic) mice, divided randomly into groups of 10 mice each, were implanted subcutaneously with five $2mm^3$ tumor fragments. Test agents (conjugates and controls) were administered intraperitoneally on days 1, 5 and 9 following the day of tumor implantation.

Developing tumors were measured (2 diameters) using vernier calipers and the mean tumor weight (wt) in mg was estimated from the following formula:

$$wt = \frac{L\ (W)^2}{2}$$

where L and W represent the length and width, in mm, of the tumor, respectively.

A treated/control (T/C) in % was calculated for groups with greater than 65% survivors, (S/T $\geq$ 6/10) and a T/C value of $\leq$42% (58% inhibition of tumor growth) was considered necessary to demonstrate activity. Cytoxan was included in each experiment as a positive control.

## Table II

**Conjugate:** (Antibody)-NH-[CO$(CH_2)_2$CONH-Gly-Gly-Asp-Ala$]_2$-Drug

**Drug:** 1,4-Bis[(2-aminoethyl)amino]-5,8-dihydroxyanthraquinone

**Antibody:** p96.5 (anti-melanoma)

**Drug-Peptide Control:** [$HO_2C(CH_2)_2$CONH-Gly-Gly-Asp-Ala$]_2$-Drug (product of example 103)

| Treatment | Per injection: Drug (mcg) | Antibody (mg) | Days post implantation, Avg. Tumor wt. in mg (T/C %): 7 | 14 | 21 | 28 | Survivors/ Treated (day 28) |
|---|---|---|---|---|---|---|---|
| Saline Control | -- | -- | 39 | 116 | 277 | 338 | 10/10 |
| Conjugate | 60 | 7.4 | 39 (100) | 57 (49) | 110 (40) | 151 (45) | 10/10 |
| Drug-Peptide Control | 60 | -- | 25 (64) | 102 (88) | 213 (77) | 404 (120) | 4/10 |
| Antibody Control | -- | 2.2 | 40 (102) | 71 (61) | 181 (65) | 424 (125) | 5/10 |

**Table II (continued)**

| Treatment | Per injection: Drug (mcg) | Per injection: Antibody (mg) | Days post implantation, Avg. Tumor wt. in mg (T/C %): 7 | 14 | 21 | 28 | Survivors/ Treated (day 28) |
|---|---|---|---|---|---|---|---|
| Drug Control | 60 | -- | 38 (97) | 56 (48) | 121 (44) | 244 (72) | 4/10 |
| | 40 | -- | 46 (118) | 81 (70) | 177 (64) | 211 (62) | 8/10 |
| Cytoxan (positive control, 80 mg/kg) | -- | -- | 45 (115) | 46 (40) | 64 (23) | -- | 0/8 |

**Table II (continued)**

**Conjugate:** (Antibody)-NH-[$CO(CH_2)_2$CONH-Asp-Ser-Ala-Leu-Leu]$_2$-Drug

HO-

**Drug:** 1,4-Bis[(2-aminoethyl)amino]-5,8-dihydroxyanthraquinone

**Antibody:** p96.5 (anti-melanoma)

**Drug-Peptide Control:** [$HO_2C(CH_2)_2$CONH-Asp-Ser-Ala-Leu-Leu]$_2$-Drug (product of example 102)

| Treatment | Per injection: Drug (mcg) | Antibody (mg) | Days post implantation, Avg. Tumor wt. in mg (T/C %): 7 | 14 | 21 | 28 | 35 | Survivors/ Treated (day 35) |
|---|---|---|---|---|---|---|---|---|
| Saline Control | -- | -- | 12 | 39 | 121 | 245 | 486 | 10/10 |
| Conjugate | 60 | 2.2 | 6 (50) | 19 (49) | 38 (31) | 89 (36) | 182 (37) | 10/10 |
| Drug-Peptide Control | 60 | -- | 22 (183) | 37 (95) | 90 (74) | 206 (84) | 413 (85) | 6/10 |
| Drug-Antibody Control | 60 | 2.2 | 12 (100) | 23 (59) | 58 (48) | 135 (55) | 266 (55) | 9/10 |

**Table II (continued)**

| Treatment | Per injection: Drug (mcg) | Antibody (mg) | Days post implantation Avg. Tumor wt. in mg (T/C %): 7 | 14 | 21 | 28 | 35 | Survivors/ Treated (day 35) |
|---|---|---|---|---|---|---|---|---|
| Drug Control | 80 | -- | 23 (192) | 27 (69) | 40 (33) | -- | -- | 0/10 |
| | 60 | -- | 12 (100) | 48 (123) | 94 (78) | 177 (72) | 348 (72) | 10/10 |
| | 40 | -- | 15 (125) | 31 (79) | 58 (48) | 143 (58) | 264 (54) | 10/10 |
| Cytoxan (Positive Control, 80 mg/kg) | -- | -- | -- | 25 (64) | 42 (35) | 54 (22) | 97 (20) | 8/8 |

The invention includes the novel compositions of matter and the method of inducing the regression and/or palliation of leukemia and related cancers in mammals using the novel compounds of this invention prior to conjugation to antibodies when administered in amounts ranging from about 1 mg to about 1.2 g per square meter of body surface area per day. The inter-relationship of dosages for animals of various sizes and species and humans (based on mg/m$^2$ of surface area) is described by Freireich, E. J., et al., Quantitative Comparison of Toxicity of Anticancer Agents in Mouse, Rat, Hamster, Dog, Monkey and Man,

Cancer Chemother. Rep., 50, 219-244 (1966). A preferred dosage regimen for optimum results would be from about 3 $mg/m^2/day$ to about 200 $mg/m^2/day$ and such dosage units are employed that a total of from about 5 mg to about 360 mg of the active compound for a subject of about 70 kg of body weight are administered in a 24 hour period. This dosage regimen may be adjusted to provide the optimum therapeutic response. For example, several divided doses may be administered daily or the dose may be proportionally reduced as indicated by the exigencies of the therapeutic situation. The active compound may be administered parenterally by the intravenous, intramuscular, or subcutaneous routes.

Solutions or dispersions of the active compound can be prepared in water suitably mixed with a surfactant such as hydroxypropylcellulose. Dispersions can also be prepared in glycerol, liquid polyethylene glycols, and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol), suitable mixtures thereof, and vegetable oils. The proper fluidity can be maintained, for example, by the use of a coating such as lecithin, by the maintenance of the required particle size in the case of a dispersion and by the use of surfactants. The prevention of the action of microorganisms can be brought about by various antibacterial and antifungal agents, for example, parabens, chlorobutanol, phenol, sorbic acid, thimerosal, and the like. In many cases it will be preferable to include isotonic agents, for example sugars or sodium chloride. Prolonged absorption of the injectable compositions can be brought about by the use in the compositions of agents delaying absorption, for example aluminum monostearate and gelatin.

Sterile injectable solutions are prepared by incorporating the active compound in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from a previously sterile-filtered solution thereof.

As used herein, "pharmaceutically acceptable carrier" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the active ingredient, its use in the therapeutic compositions is contemplated. Supplementary active ingredients can also be incorporated into the compositions.

It is especially advantageous to formulate parenteral compositions in dosage unit form for ease of administration and uniformity of dosage. Dosage unit form as used herein refers to physically discrete units suited as unitary dosages for the mammalian subject to be treated; each unit contains a predetermined quantity of active material calculated to produce the desired therapeutic effect in association with the required pharmaceutical carrier. The specification for the novel dosage unit forms of the invention are dictated by and directly dependent on the unique characteristics of the active material, the particular therapeutic effect to be achieved and the limitations inherent in the art of compounding such an active material for the treatment of disease in living subjects having a diseased condition in which bodily health is impaired as herein disclosed in detail.

The principal active ingredient is compounded for convenient and effective administration in effective amounts with a suitable pharmaceutically acceptable carrier in dosage unit form as hereinbefore disclosed. A unit dosage form can, for example, contain the principal active compound in amounts ranging from about 2 mg to about 2 g, with from about 5 mg to about 360 mg being preferred. Expressed in proportions, the active compound is generally present in from about 2 to about 100 mg/ml of carrier. In the case of compositions containing supplementary active ingredients, the dosages are determined by reference to the usual dose and manner of administration of the said ingredients.

Likewise, the novel antibody conjugates of the invention are useful in the regression, palliation, and treatment of cancers, and due to their primarily proteinaceous nature, are preferably prepared for use in formulations suitable for injection. Vacuum dried or freeze-dried preparations are likewise contemplated if

such techniques of formulation do not significantly alter the antigen recognition capabilities of the conjugates. Thus the invention also includes pharmaceutical formulations of antibody conjugates suitable for injection, comprising dosage units from about 3 $mg/m^2$/day to about 2 $g/m^2$/day of mammalian body surface area of a conjugate of the invention together with a pharmaceutically-acceptable carrier, diluent, stabilizer, and/or antibacterial or antifungal agent as described previously. Preferable unit dosage forms of the conjugates are contemplated to contain from about 0.1 mg to about 200 mg of the active anthraquinone ingredient. However, it will be understood that the amount of the conjugate actually administered will be determined by a physician in the light of relevant circumstances, including the tumor to be treated, age of the patient, advanced nature of the disease, chosen route of administration, and the like.

Regression and palliation of cancers are attained, for example, using intraperitoneal administration. A single intravenous dosage or repeated daily dosages can be administered. Daily dosages for up to about 5 to 10 days are often sufficient. It is also possible to dispense one daily dosage or one dose on alternate or less frequent days. As can be seen from the dosage regimens, the amount of principal active ingredient administered is a sufficient amount to aid regression and palliation of the leukemia or the like, in the absence of excessive deleterious side effects of a cytotoxic nature to the host harboring the cancer. As used herein, cancer disease means blood malignancies such as leukemia, as well as other solid and non-solid malignancies such as the melanocarcinomas, lung carcinomas and mammary tumors. By regression and palliation is meant arresting or retarding the growth of the tumor or other manifestation of the disease compared to the course of the disease in the absence of treatment.

This invention will be described in greater detail in conjunction with the following non-limiting specific examples.

Example 1

**[S-(R*,R*)]-N,N'-[(9,10-Dihydro-5,8-dihydroxy-9,10-dioxo-1,4-anthracenediyl)bis[imino-2,1-ethanediylimino-(1-methyl-2-oxo-2,1-ethanediyl)]]bis-carbamic acid, bis(1,1-dimethylethyl)ester**

A 1.78 g portion of 1,4-bis[(2-aminoethyl) amino]-5,8-dihydroxyanthraquinone, dihydrochloride (U. S. Patent 4,526,989) and 2.53 g of triethylamine were slurried in 50 ml of dry tetrahydrofuran, then cooled in an ice-methanol bath. A solution of 2.72 g of trimethyl silyl chloride in 20 ml of dry tetrahydrofuran was added dropwise with stirring. The mixture was stirred in the ice bath for 40 minutes, then at room temperature for 40 minutes and then filtered. The filtrate was cooled in an ice-methanol bath and a solution of 3.15 g of N-tertiarybutyloxycarbonyl-L-alanine hydroxysuccinimide ester in 50 ml of dry tetrahydrofuran was added dropwise with stirring. The ice bath was allowed to melt, then the reaction was stirred for 24 hours and finally filtered. The filtrate was passed through a silica gel pad, washed with 500 ml of dry tetrahydrofuran and then concentrated in vacuo at 60°C. The residue was slurried in 50 ml of ethyl acetate, filtered and dried in vacuo giving 584 mg of the desired product, mp 195°C (dec.).

Example 2

**[S-(R*,R*)]-N,N'-[(9,10-Dihydro-5,8-dihydroxy-9,10-dioxo-1,4-anthracenediyl)bis[imino-2,1-ethanediylimino-[1-(2-methylpropyl)-2-oxo-2,1-ethanediyl]]]bis-carbamic acid, bis(1,1-dimethylethyl)ester**

The general procedure of Example 1 was followed using 3.61 g of N-tertiarybutyloxycarbonyl-L-leucine hydroxysuccinimide ester in place of the L-alanine ester and appropriate amounts of the other reagents, giving 713 mg of the desired product, mp 178°C (dec.).

Example 3

**[R-(R*,R*)]-N,N'-[(9,10-Dihydro-5,8-dihydroxy-9,10-dioxo-1,4-anthracenediyl)bis(imino-2,1-ethanediyliminocarbonylethylidene)]bis-carbamic acid, bis(1,1-dimethylethyl)ester**

The general procedure of Example 1 was followed using 3.15 g of N-tertiarybutyloxycarbonyl-D-alanine hydroxysuccinimide ester and appropriate amounts of the other reagents, giving 900 mg of the desired compound, mp 198°C (dec.).

Example 4

**[S-(R*,R*)]-N,N'-[(9,10-Dihydro-5,8-dihydroxy-9,10-dioxo-1,4-anthracenediyl)bis(imino-2,1-ethanediyl)]bis-[2-aminopropanamide], dihydrochloride**

A 485 mg portion of [S-(R*,R*)]-N,N'-[(9,10-dihydro-5,8-dihydroxy-9,10-dioxo-1,4-anthracenediyl)bis-[imino-2,1-ethanediylimino(1-methyl-2-oxo-2,1-ethanediyl)]]bis-carbamic acid, bis(1,1-dimethylethyl)ester was slurried in 10 ml of glacial acetic acid and 5 ml of anisole was added. Hydrogen chloride gas was bubbled into the solution for 5 minutes with stirring, then the mixture was allowed to stand overnight. The resulting gum was stirred with 30 ml of ether giving a solid which was collected, washed with ether and dried in vacuo at 80°C, to give 8 mg of the desired product. High performance liquid chromatography ("HPLC") retention time on a Whatman column using water:acetonitrile:methyl cellosolve:formic acid at the indicated ratio at a flow rate of 2.5 ml/minute here and in future examples:
75:20:2.5:2.5 = 209 seconds

The subject compound was also obtained as the bis-trifluoroacetate salt when the starting material was treated with trifluoroacetic acid-anisole by the procedure described in following Example 20.

Example 5

**[S-(R*,R*)]-N,N'-[(9,10-Dihydro-5,8-dihydroxy-9,10-dioxo-1,4-anthracenediyl)bis(imino-2,1-ethanediyl)]bis-[2-amino-4-methylpentanamide], dihydrochloride**

A 1.50 g portion of [S-(R*,R*)]-N,N'-[(9,10-dihydro-5,8-dihydroxy-9,10-dioxo-1,4-anthracenediyl)bis[imino-2,1-ethanediylimino[1-(2-methylpropyl)-2-oxo-2,1-ethanediyl]]]bis-carbamic acid, bis(1,1-dimethylethyl)ester was reacted as described in Example 4, giving 867 mg of the desired product as a blue-black solid, mp 180-183°C.

The subject compound was obtained as the bis-trifluoroacetate salt when the staring material was treated with trifluoroacetic acid-anisole by the procedure described in following Example 20.

Example 6

**[R-(R*,R*)]-N,N'-[(9,10-Dihydro-5,8-dihydroxy-9,10-dioxo-1,4-anthracenediyl)bis(imino-2,1-ethanediyl)]bis-[2-aminopropanamide], dihydrochloride**

A 700 mg portion of [R-(R*,R*)]-N,N'-[(9,10-dihydro-5,8-dihydroxy-9,10-dioxo-1,4-anthracenediyl)bis-(imino-2,1-ethanediyliminocarbonylethylidene)]bis-carbamic acid, bis(1,1-dimethylethyl)ester was reacted as described in Example 4, giving 542 mg of the desired product, HPLC 70:20:5:5 = 196 seconds.

Example 7

**[S-(R*,R*)]-N,N'-[(9,10-Dihydro-5,8-dihydroxy-9,10-dioxo-1,4-anthracenediyl)bis[imino-2,1-ethanediylimino[1-(2-methylpropyl)-2-oxo-2,1-ethanediyl]]]bis-carbamic acid, (phenylmethyl)ester**

A 1.78 g portion of 1,4-bis[(2-aminoethyl) amino]-5,8-dihydroxyanthraquinone, dihydrochloride and 3.48 ml of triethylamine were slurried in 50 ml of tetrahydrofuran cooled in an ice bath. A solution of 3.72 g of trimethylsilyl chloride in 20 ml of tetrahydrofuran was added dropwise. The mixture was stirred for one hour in the ice bath, then for one hour at room temperature and filtered. To the filtrate was added 4.0 g of carbobenzyloxy-L-leucine hydroxysuccinimide ester. The mixture was stirred overnight and then filtered. The filtrate was passed through a silica pad, then eluted with 500 ml of tetrahydrofuran and the eluent concentrated in vacuo. The residue was slurried in 50 ml of ethyl acetate, the solid collected, washed with ether and dried in vacuo, giving 1.0 g of the desired product, mp 180°C (dec.).

Example 8

**[S-(R*,R*)]-N,N'-[(9,10-Dihydro-5,8-dihydroxy-9,10-dioxo-1,4-anthracenediyl)bis(imino-2,1-ethanediyl)]-bis[2-amino-4-methylpentanamide], dihydrobromide**

A 500 mg portion of [S-(R*,R*)]-N,N'-[(9,10-dihydro-5,8-dihydroxy-9,10-dioxo-1,4-anthracenediyl)bis-[imino-2,1-ethanediylimino[1-(2-methylpropyl)-2-oxo-2,1-ethanediyl]]]bis-carbamic acid, (phenylmethyl)ester in 10 ml of glacial acetic acid was cooled in an ice bath to just above freezing. Anhydrous hydrogen bromide was bubbled in for 5 minutes, then the mixture was stirred at room temperature overnight. After dilution with 50 ml of ether, the solid was collected, washed with ether and dried in vacuo, giving 460 mg of the desired product, HPLC 60:32:4:4 = 161 seconds.

Example 9

**[S-(R*,R*)]-N,N'-[(9,10-Dihydro-5,8-dihydroxy-9,10-dioxo-1,4-anthracenediyl)bis[imino-2,1-ethanediylimino-(1-oxo-1,2,6-hexanetriyl)]]tetrakis-carbamic acid, tetrakis(phenylmethyl)ester**

A mixture of 890 mg of 1,4-bis[(2-aminoethyl)amino]-5,8-dihydroxyanthraquinone, dihydrochloride, and 2.8 g of bis-(carbonylbenzyloxy)-L-lysine hydroxysuccinimide ester in 50 ml of dimethylformamide was stirred overnight and then filtered. The insolubles were slurried with 120 ml of dimethylformamide and filtered. The filtrates were combined, concentrated in vacuo and the residue slurried in 150 ml of tetrahydrofuran. The solid was collected, washed with ether and dried in vacuo, giving 1.3 g of the desired product, mp >200°C.

Example 10

**[S-(R*,R*)]-4,4'-[(9,10-Dihydro-5,8-dihydroxy-9,10-dioxo-1,4-anthracenediyl)bis(imino-2,1-ethanediylimino)]bis[3-[[9H-fluoren-9-ylmethoxycarbonyl]amino]-4-oxobutanoic acid], bis(1,1-dimethylethyl)ester**

A 1.029 g portion of fluorenylmethyloxycarbonyl aspartic acid, β-tertiary butyl ester was slurried in 30 ml of ethyl acetate. A 284 mg portion of N-methylmorpholine was added and the mixture was cooled in an ice-methanol bath. A 382 mg portion of isobutyloxycarbonyl chloride was added, the mixture was stirred for 5 minutes, then 322 mg of N-hydroxysuccinimide in 1 ml of dimethylformamide was added. The mixture was stirred 5 minutes in the cold, then at room temperature overnight, diluted with 50 ml of ethyl acetate, cooled in an ice bath and extracted with two 10 ml portions of ice water. The extracts were combined, dried with sodium sulfate, evaporated to an oil and dissolved in 15 ml of dimethylformamide. This solution was added to a solution of 300 mg of 1,4-bis-[(2-aminoethyl)amino]-5,8-dihydroxyanthraquinone in 10 ml of dimethylformamide. This mixture was stirred overnight, then evaporated to dryness, giving 707 mg of [S-(R*,R*)]-4,4'-[(9,10-dihydro-5,8-dihydroxy-9,10-dioxo-1,4-anthracenediyl)bis(imino-2,1-ethanediylimino)]bis[3-[[(9H-fluoren-9-ylmethoxy)carbonyl]amino]-4-oxobutanoic acid] bis(1,1-dimethylethyl)ester.

Example 11

**[S-(R*,R*)]-4,4'-[(9,10-Dihydro-5,8-dihydroxy-9,10-dioxo-1,4-anthracenediyl)bis(imino-2,1-ethanediylimino)]bis[3-amino-4-oxobutanoic acid] bis(1,1-dimethylethyl)ester**

A 350 mg portion of the product of Example 10 was dissolved in 20 ml of dimethylformamide. Dimethylamine was bubbled into the solution for 5 minutes, then the mixture was evaporated to dryness, triturated with ether and dried, giving 208 mg of the desired product, mp 140°C (dec.).

Example 12

**N,N'-[(9,10-Dihydro-5,8-dihydroxy-9,10-dioxo-1,4-anthracenediyl)bis(imino-2,1-ethanediyl)][N-[(phenylmethoxy)carbonyl]-L-leucyl-L-alaninamide]**

A mixture of 36 mg of 1,4-bis[(2-aminoethyl)amino]-5,8-dihydroxyanthraquinone and 100 mg of N-carbobenzyloxy-L-leucyl-L-alanyl hydroxysuccinimide ester in 2.5 ml of dimethylformamide was stirred overnight, then evaporated to dryness. The residue was slurried in 100 ml of methanol, filtered, slurried in 10 ml of water at pH 1.8 for 1/2 hour, collected by filtration and dried. The residue was slurried in ethyl acetate, then evaporated to dryness, giving 48 mg of the desired product, mp >220°C (dec.).

Example 13

**N,N'-[(9,10-Dihydro-5,8-dihydroxy-9,10-dioxo-1,4-anthracenediyl)bis(imino-2,1-ethanediyl)]bis[L-leucyl-L-alaninamide], dihydrobromide**

A 34 mg portion of N,N'-[(9,10-dihydro-5,8-dihydroxy-9,10-dioxo-1,4-anthracenediyl)bis(imino-2,1-ethanediyl)][N-[(phenylmethoxy)carbonyl]-L-leucyl-L-alaninamide] in 5 ml of glacial acetic acid saturated with hydrogen bromide was stirred overnight, precipitated with ether, collected and dried, giving 32 mg of the desired product, mp 200°C (dec.).

Example 14

**N,N'-[(9,10-Dihydro-5,8-dihydroxy-9,10-dioxo-1,4-anthracenediyl)bis(imino-2,1-ethanediyl)]bis[N-[(phenylmethoxy)carbonyl]-L-leucyl-L-alanyl-L-leucinamide]**

A mixture of 199 mg of [S-(R*,R*)]-N,N'-[(9,10-dihydro-5,8-dihydroxy-9,10-dioxo-1,4-anthracenediyl)bis-(imino-2,1-ethanediyl)]bis[2-amino-4-methylpentanamide], dihydrobromide, 229 mg of carbobenzyloxy-L-leucyl-L-alanyl hydroxysuccinimide ester and 0.2 ml of triethylamine in 10 ml of dimethylformamide was stirred overnight and then evaporated to dryness. The residue was triturated with ethyl acetate and then filtered. The filtrate was washed twice with a mixture of 50 ml of 0.1N hydrochloric acid and 50 ml of water, then evaporated giving 225 mg of the desired product, mp >250°C.

Example 15

**N,N'-[(9,10-Dihydro-5,8-dihydroxy-9,10-dioxo-1,4-anthracenediyl)bis(imino-2,1-ethanediyl)]bis[L-leucyl-L-alanyl-L-leucinamide] dihydrobromide**

A 75 mg portion of N,N'-[(9,10-dihydro-5,8-dihydroxy-9,10-dioxo-1,4-anthracenediyl)bis(imino-2,1-ethanediyl)]bis[N-[(phenylmethoxy)carbonyl]-L-leucyl-L-alanyl-L-leucinamide was reacted as described in Example 6, giving 68 mg of the desired product, mp 195°C (dec.).

Example 16

**N,N'-[9,10-Dihydro-5,8-dihydroxy-9,10-dioxo-1,4-anthracenediyl)bis(imino-2,1-ethanediyl)]bis[N-[phenylmethoxy)carbonyl]glycyl-L-phenylalaninamide]**

A 7.17 g portion of carbobenzyloxy glycyl-L-phenylalanine and 2.30 g of N-hydroxysuccinimide were slurried in 100 ml of acetonitrile, cooled in an ice bath, with the addition of 5 ml of dimethylformamide, to obtain solution. A precooled solution of 4.12 g of dicyclohexylcarbodiimide in 50 ml of acetonitrile was added dropwise. The mixture was stirred in the ice bath for 2 hours, then refrigerated overnight, filtered and the filtrate concentrated in vacuo. The residue was taken up in 125 ml of ethyl acetate, washed with precooled 5% aqueous sodium bicarbonate, then twice with water, dried and concentrated in vacuo. The residue was dissolved in 125 ml of 2-propanol and refrigerated overnight. The resulting gum was taken up in tetrahydrofuran and concentrated in vacuo, giving 6.5 g of 1-[[N-[N-[(phenylmethoxy)carbonyl]glycyl]-L-phenylalanyl]oxy]-2,5-pyrrolidinedione.

A mixture of 2.4 g of the above ester was reacted as described in Example 9, giving 560 mg of the desired product, mp >200°C.

Example 17

**N,N'-[(9,10-Dihydro-5,8-dihydroxy-9,10-dioxo-1,4-anthracenediyl)bis(imino-2,1-ethanediyl)]bis[N-[(phenylmethoxy)carbonyl]glycyl]glycinamide]**

A 7.98 g portion of carbobenzyloxy glycylglycine was reacted as described in Example 16, giving 6.15 g of 1-[[N-[N-[(phenylmethoxy)carbonyl]glycyl]glycyl]oxy]-2,5-pyrrolidinedione.

A 4.18 g portion of the above ester was reacted as described in Example 14, giving 3.4 g of the desired product, mp 155°C (dec.).

Example 18

**N,N'-[(9,10-Dihydro-5,8-dihydroxy-9,10-dioxo-1,4-anthracenediyl)bis(imino-2,1-ethanediyl)]bis[glycylglycinamide] dihydrobromide**

A 3 g portion of N,N'-[(9,10-dihydro-5,8-dihydroxy-9,10-dioxo-1,4-anthracenediyl)bis(imino-2,1-ethanediyl)]bis[N-[(phenylmethoxy)carbonyl]glycyl]glycinamide was reacted as described in Example 8, giving 3.4 g of the desired product, mp 100°C (dec.).

Example 19

**N,N'-[(9,10-Dihydro-5,8-dihydroxy-9,10-dioxo-1,4-anthracenediyl)bis(imino-2,1-ethanediyl)]bis[N-[(1,1-dimethylethoxy)carbonyl]glycylglycyl]glycinamide**

A mixture of 2.6 g of N,N'-[(9,10-dihydro-5,8-dihydroxy-9,10-dioxo-1,4-anthracenediyl)bis(imino-2,1-ethanediyl)]bis[glycylglycinamide] dihydrobromide, 1.8 g of tertiary butyloxycarbonyl glycine hydroxysuccinimide ester and 2.6 ml of triethylamine in 100 ml of dimethylformamide was stirred for 2 days, then evaporated to dryness. The residue was triturated with ethyl acetate, filtered and dried in vacuo. This residue was triturated in 100 ml of cold water at pH 3 for 1/2 hour, then filtered and the solid dried, giving 1.8 g of the desired product, mp 165°C (dec.).

Example 20

N,N'-[(9,10-Dihydro-5,8-dihydroxy-9,10-dioxo-1,4-anthracenediyl)bis(imino-2,1-ethanediyl]bis[[glycylglycyl]-glycinamide] bis(trifluoroacetate)

A 1.5 g portion of N,N'-[(9,10-dihydro-5,8-dihydroxy-9,10-dioxo-1,4-anthracenediyl)bis(imino-2,1-ethanediyl)]bis[[N-[(1,1-dimethylethoxy)carbonyl]glycylglycyl]glycinamide] was slurried in 5 ml of anisole, cooled in an ice bath and 10 ml of trifluoroacetic acid was added. The mixture was stirred in the ice bath for 15 minutes, then at room temperature for 1.5 hours and diluted with 50 ml of ether. The solid was collected, washed with ether and dried in vacuo, giving 1.69 g of the desired product, mp. 100-113°C.

Example 21

**N,N'-[(9,10-Dihydro-5,8-dihydroxy-9,10-dioxo-1,4-anthracenediyl)bis(imino-2,1-ethanediyl)]bis[N-[(1,1-dimethylethoxy)carbonyl]-L-alanyl-L-leucinamide]**

A mixture of 3.25 g of [S-(R*,R*)]-N,N'-[(9,10-dihydro-5,8-dihydroxy-9,10-dioxo-1,4-anthracenediyl)bis-(imino-2,1-ethanediyl)]bis[2-amino-4-methylpentanamide], bis(trifluoroacetate), prepared from the compound of Example 2 by trifluoroacetic acid cleavage of the protecting group, 3.45 ml of triethylamine and 125 ml of dimethylformamide was stirred and 2.52 g of N-tertiary-butyloxycarbonyl-L-alanine hydroxysuccinimide ester was added. The mixture was stirred overnight, then evaporated to dryness, washed with 500 ml of ethyl acetate, air-dried and washed then with 250 ml of water. The solid was collected, giving 2.3 g of the desired product, mp 215°C (dec.).

Example 22

**N,N'-[(9,10-Dihydro-5,8-dihydroxy-9,10-dioxo-1,4-anthracenediyl)bis(imino-2,1-ethanediyl)]bis-[L-alanyl-L-leucinamide] bis(trifluoroacetate)**

A 2 g portion of N,N'-[(9,10-dihydro-5,8-dihydroxy-9,10-dioxo-1,4-anthracenediyl)bis(imino-2,1-ethanediyl)]bis[N-[(1,1-dimethylethoxy)carbonyl]-L-alanyl-L-leucinamide] was reacted as described in Example 20, giving 2.05 g of the desired compound, mp 185°C (dec.).

Example 23

**N,N'-[(9,10-Dihydro-5,8-dihydroxy-9,10-dioxo-1,4-anthracenediyl)bis(imino-2,1-ethanediyl)]bis[N-[1,1-dimethylethoxy)carbonyl]-L-alanyl-L-alanyl-L-leucinamide]**

A 953 mg portion of N,N'-[(9,10-dihydro-5,8-dihydroxy-9,10-dioxo-1,4-anthracenediyl)bis(imino-2,1-ethanediyl)]bis[L-alanyl-L-leucinamide] bis(trifluoroacetate) and 630 mg of N-tertiarybutyloxycarbonyl-L-alanine hydroxysuccinimide ester were reacted as described in Example 21, giving 907 mg of the desired product, mp >220°C.

Example 24

**N,N'-[(9,10-Dihydro-5,8-dihydroxy-9,10-dioxo-1,4-anthracenediyl)bis(imino-2,1-ethanediyl)]bis[L-alanyl-L-alanyl-L-leucinamide] bis(trifluoroacetate)**

A 700 mg portion of N,N'-[(9,10-dihydro-5,8-dihydroxy-9,10-dioxo-1,4-anthracenediyl)bis(imino-2,1-ethanediyl)]bis[N-[(1,1-dimethylethoxy)carbonyl]-L-alanyl-L-alanyl-L-leucinamide] was reacted as described in Example 20, giving 860 mg of the desired product, mp >210°C.

Example 25

**N,N'-[(9,10-Dihydro-5,8-dihydroxy-9,10-dioxo-1,4-anthracenediyl)bis(imino-2,1-ethanediyl)]bis[N-[(phenylmethoxy)carbonyl]glycylglycyl-L-leucinamide]**

A 1.23 g portion of [S-(R*,R*)]-N,N'-[(9,10-dihydro-5,8-dihydroxy-9,10-dioxo-1,4-anthracenediyl)bis(imino-2,1-ethanediyl)]bis[2-amino-4-methylpentanamide] bis(trifluoroacetate) and 1.19 g of carbobenzyloxyglycylglycyl hydroxysuccinimide ester were reacted as described in Example 21, giving 1.28 g of the desired compound, mp 197°C (dec.).

Example 26

**N,N'-[(9,10-Dihydro-5,8-dihydroxy-9,10-dioxo-1,4-anthracenediyl)bis(imino-2,1-ethanediyl)]bis[glycylglycyl-L-leucinamide] dihydrobromide**

A 1 g portion of N,N'-[(9,10-dihydro-5,8-dihydroxy-9,10-dioxo-1,4-anthracenediyl)bis(imino-2,1-ethanediyl)]bis[N-[(phenylmethoxy)carbonyl]glycylglycyl-L-leucinamide] was reacted as described in Example 8, giving 1.01 g of the desired product, mp 189°C (dec.).

Example 27

**N,N'-[(9,10-Dihydro-5,8-dihydroxy-9,10-dioxo-1,4-anthracenediyl)bis(imino-2,1-ethanediyl)]bis[N-[(1,1-dimethylethoxy)carbonyl]-L-alanyl-L-alaninamide]**

A 2.4 g portion of [S-(R*,R*)]-N,N'-[(9,10-dihydro-5,8-dihydroxy-9,10-dioxo-1,4-anthracenediyl)bis(imino-2,1-ethanediyl)]bis[2-aminopropanamide] bis(trifluoroacetate) and 2.07 g of N-tertiarybutyloxycarbonyl-L-alanine hydroxysuccinimide ester were reacted as described in Example 21, giving 1.8 g of the desired product, mp >200°C (dec.).

Example 28

**N,N'-[(9,10-Dihydro-5,8-dihydroxy-9,10-dioxo-1,4-anthracenediyl)bis(imino-2,1-ethanediyl)]bis-[L-alanyl-L-alaninamide] bis(trifluoroacetate)**

A 1.5 g portion of N,N'-[(9,10-dihydro-5,8-dihydroxy-9,10-dioxo-1,4-anthracenediyl)bis(imino-2,1-ethanediyl)]bis[N-[(1,1-dimethylethoxy)carbonyl]-L-alanyl-L-alaninamide] was reacted as described in Example 20, giving 1.52 g of the desired product, mp 175°C (dec.).

Example 29

**N,N'-[(9,10-Dihydro-5,8-dihydroxy-9,10-dioxo-1,4-anthracenediyl)bis(imino-2,1-ethanediyl)]bis[N-[(1,1-dimethylethoxy)carbonyl]-L-alanyl-L-alanyl-L-alaninamide]**

An 870 mg portion of N,N'-[(9,10-dihydro-5,8-dihydroxy-9,10-dioxo-1,4-anthracenediyl)bis(imino-2,1-

ethanediyl)]bis[L-alanyl-L-alaninamide] bis(trifluoroacetate) and 629 mg of N-tertiarybutyloxycarbonyl-L-alanine hydroxysuccinimide ester were reacted as described in Example 21, giving 890 mg of the desired product, mp >200°C (dec.).

Example 30

**N,N'-[(9,10-Dihydro-5,8-dihydroxy-9,10-dioxo-1,4-anthracenediyl)bis(imino-2,1-ethanediyl)]bis[L-alanyl-L-alanyl-L-alaninmide] bis(trifluoroacetate)**

A 600 mg portion of N,N'-[(9,10-dihydro-5,8-dihydroxy-9,10-dioxo-1,4-anthracenediyl)bis(imino-2,1-ethanediyl)]bis[N-[(1,1-dimethylethoxy)carbonyl]-L-alanyl-L-alanyl-L-alaninamide] was reacted as described in Example 20, giving 614 mg of the desired product, mp 190°C (dec.).

Example 31

**N,N'-[(9,10-Dihydro-5,8-dihydroxy-9,10-dioxo-1,4-anthracenediyl)bis(imino-2,1-ethanediyl)]bis[N-[(1,1-dimethylethoxy)carbonyl]-L-seryl-L-alaninamide**

A 727 mg portion of [S-(R*,R*)]-N,N'-[(9,10-dihydro-5,8-dihydroxy-9,10-dioxo-1,4-anthracenediyl)bis-(imino-2,1-ethanediyl)]bis[2-aminopropanamide] bis(trifluoroacetate) and 665 mg of N-tertiarybutyloxycarbonyl-L-serine hydroxysuccinimide ester were reacted as described in Example 21, giving 619 mg of the desired product, mp >220°C (dec.).

Example 32

**N,N'-[(9,10-Dihydro-5,8-dihydroxy-9,10-dioxo-1,4-anthracenediyl)bis(imino-2,1-ethanediyl)]bis-[L-seryl-L-alaninamide] bis(trifluoroacetate)**

A 419 mg portion of N,N'-[(9,10-dihydro-5,8-dihydroxy-9,10-dioxo-1,4-anthracenediyl)bis(imino-2,1-ethanediyl)]bis[N-[(1,1-dimethylethoxy)carbonyl]-L-seryl-L-alaninamide] was reacted as described in Example 20, giving 386 mg of the desired product, mp 130°C (dec.).

Example 33

**N,N'-[(9,10-Dihydro-5,8-dihydroxy-9,10-dioxo-1,4-anthracenediyl)bis(imino-2,1-ethanediyl)]bis[N-[(9H-fluoren-9-ylmethoxy)carbonyl]-L-α-aspartyl-L-seryl-L-alaniamide] bis(1,1-dimethylethyl)ester**

A 300 mg portion of N,N'-[(9,10-dihydro-5,8-dihydroxy-9,10-dioxo-1,4-anthracenediyl)bis(imino-2,1-ethanediyl)]bis[L-seryl-L-alaninamide], bis(trifluoroacetate) and 0.75 mmole of N-[(9H-fluoren9-ylmethoxy)-carbonyl]-L-α-aspartic acid hydroxysuccinimide ester, γ-O-(1,1-dimethylethyl)ester (prepared as in Example 10) were reacted as described in Example 10, giving 363 mg of the desired product, mp 195°C (dec.).

Example 34

**N,N'-[(9,10-Dihydro-5,8-dihydroxy-9,10-dioxo-1,4-anthracenediyl)bis(imino-2,1-ethanediyl)]bis[N-[(9H-fluoren-9-ylmethoxy)carbonyl]-L-α-aspartyl-L-alanyl-L-alaninamide] bis(1,1-dimethylethyl)ester**

A 1.74 g portion of N,N'-[(9,10-dihydro-5,8-dihydroxy-9,10-dioxo-1,4-anthracenediyl)bis(imino-2,1-ethanediyl)]bis[L-alanyl-L-alaninamide] bis(trifluoro-acetate) and 4.4 mmole of N-[(9H-fluoren-9-ylmethoxy)-carbonyl]-L-α-aspartic acid hydroxysuccinimide ester, γ-O-(1,1-dimethylethyl)ester (prepared as in Example 10) were reacted as described in Example 10, giving 2.28 g of the desired product, mp 215°C (dec.).

Example 35

**N,N'-[(9,10-Dihydro-5,8-dihydroxy-9,10-dioxo-1,4-anthracenediyl)bis(imino-2,1-ethanediyl)]bis[L-α-aspartyl-L-alanyl-L-alaninamide] bis(1,1-dimethylethyl)ester**

A 500 mg portion of N,N'-[(9,10-dihydro-5,8-dihydroxy-9,10-dioxo-1,4-anthracenediyl)bis(imino-2,1-ethanediyl)]bis[N-[(9H-fluoren-9-ylmethoxy)carbonyl]-L-α-aspartyl-L-alanyl-L-alaninamide] bis(1,1-dimethylethyl)ester was dissolved in 20 ml of N,N-dimethylformamide. Dimethylamine was bubbled into the solution for 5 minutes, then the mixture was evaporated to dryness, triturated with ether and dried giving 317 mg of the desired product, mp 208°C (dec.).

Example 36

**N,N'-[(9,10-Dihydro-5,8-dihydroxy-9,10-dioxo-1,4-anthracenediyl)bis(imino-2,1-ethanediyl)]bis[N-[(9H-fluoren-9-ylmethoxy)carbonyl]-L-α-aspartyl-L-alanyl-L-leucinamide] bis(1,1-dimethylethyl)ester**

A 1.9 g portion of N,N'-[(9,10-dihydro-5,8-dihydroxy-9,10-dioxo-1,4-anthracenediyl)bis(imino-2,1-ethanediyl)]bis[L-alanyl-L-leucinamide] bis(trifluoroacetate) and 4.4 mmole of N-[(9H-fluoren-9-ylmethoxy)-carbonyl-L-α-aspartic acid hydroxysuccinimide ester, γ-O-(1,1-dimethylethyl)ester (prepared as in Example 10) were reacted as described in Example 10, giving 2.6 g of the desired product, mp 200°C (dec.).

Example 37

**N,N'-[(9,10-Dihydro-5,8-dihydroxy-9,10-dioxo-1,4-anthracenediyl)bis(imino-2,1-ethanediyl)]bis[L-α-aspartyl-L-alanyl-L-leucinamide] bis(1,1-dimethylethyl)ester**

A 1 g portion of N,N'-[(9,10-dihydro-5,8-dihydroxy-9,10-dioxo-1,4-anthracenediyl)bis(imino-2,1-ethanediyl)]bis[N-[(9H-fluoren-9-ylmethoxy)carbonyl]-L-α-aspartyl-L-alanyl-L-leucinamide] bis(1,1-dimethylethyl)ester was reacted with dimethylamine-dimethylformamide as described in Example 11, giving 607 mg of the desired product, mp 210°C (dec.).

Example 38

**N,N'-[(9,10-Dihydro-5,8-dihydroxy-9,10-dioxo-1,4-anthracenediyl)bis(imino-2,1-ethanediyl)]bis[L-arginine(phenylmethyl)]ester, dihydrobromide**

A 1 g portion of α-carbobenzyloxy-L-arginine hydrobromide and 398 mg of hydroxybenzotriazole were dissolved in 10 ml of dimethylformamide chilled in a methanol-ice bath over 15 minutes. A 536 mg portion of dicyclohexylcarbodiimide was dissolved in a few ml of dimethylformamide and added to the mixture. The mixture was stirred one hour at 0°C, then one hour at room temperature, then filtered and the filtrate added to a solution of 338 mg of 1,4-bis[(2-aminoethyl)amino]-5,8-dihydroxyanthraquinone in dimethylformamide. This mixture was stirred overnight, then evaporated and the residue triturated with ethyl acetate, giving 1.08 g of product, Rf 0.52 in a 3:1:1 (butanol:acetic acid:water) thin layer chromatography ("tlc") system, mp 120°C (dec.).

Example 39

**N,N'-[(9,10-Dihydro-5,8-dihydroxy-9,10-dioxo-1,4-anthracenediyl)bis(imino-2,1-ethanediyl)]bis-L-argininamide, dihydrobromide**

A 900 mg portion of the compound of Example 38 was dissolved in 25 ml of glacial acetic acid saturated with hydrogen bromide, stirred for 1.5 hours, then quenched in ether and the solid collected and dried to yield 571 mg of crude product, 300 mg of which was resubjected to glacial acetic acid-saturated hydrogen bromide for 0.5 hours to yield 250 mg of product, Rf 0.1 in a tetrahydrofuran:water:acetic acid 13:2:1 tlc system.

Example 40

**N,N'-[(9,10-Dihydro-5,8-dihydroxy-9,10-dioxo-1,4-anthracenediyl)bis(imino-2,1-ethanediyl)]bis[L-alanyl-L-argininamide] bis(1,1-dimethylethyl) ester, dihydrobromide**

A 100 mg portion of the compound of Example 39 was dissolved in dimethylformamide and to it was added a solution of 63 mg of N-tertiarybutyloxycarbonyl-L-alanine hydroxysuccinimide ester and 0.086 ml of triethylamine in dimethylformamide. This mixture was stirred overnight, then evaporated and the residue triturated with ethyl acetate, giving 108 mg of product with an Rf of 0.75 in the tlc system of Example 39.

Example 41

**N,N'[(9,10-Dihydro-5,8-dihydroxy-9,10-dioxo-1,4-anthracenediyl)bis(imino-2,1-ethanediyl)]bis-[L-alanyl-L-argininamide] dihydrobromide bis(trifluoroacetate)**

A 1.55 g portion of the compound, prepared as described in Example 40, was reacted as described in Example 20, giving 1.2 g of the desired product, Rf 0.5 in tlc system A (dimethylformamide:methyl cellosolve:tetrahydrofuran:isopropanol:acetonitrile:ammonium hydroxide:acetic acid 4:3:3:2:2:1:1).

Example 42

**N,N'-[(9,10-Dihydro-5,8-dihydroxy-9,10-dioxo-1,4-anthracenediyl)bis(imino-2,1-ethanediyl)]bis-[N-[(1,1-dimethylethoxy)carbonyl]-L-alanyl-L-alanyl-L-argininamide] dihydrobromide**

A 1.0 g portion of N,N'-[(9,10-dihydro-5,8-dihydroxy-9,10-dioxo-1,4-anthracenediyl)bis(imino-2,1-ethanediyl)]bis[L-alanyl-L-argininamide] dihydrobromide bis(trifluoroacetate) was dissolved in 90 ml of dimethylformamide. A 0.69 ml portion of triethylamine was added, then 500 mg of N-tertiarybutyloxycarbonyl-L-alanine hydroxysuccinimide ester. The mixture was stirred overnight, then evaporated. The residue was triturated with ethyl acetate, then with acetone, filtered and dried *in vacuo*, giving 880 mg of the desired product, mp 200°C (dec.).

Example 43

**N,N'-[(9,10-Dihydro-5,8-dihydroxy-9,10-dioxo-1,4-anthracenediyl)bis(imino-2,1-ethanediyl)]bis[L-alanyl-L-argininamide], dihydrobromide, bis(trifluoroacetate)**

A 290 mg portion of N,N'-[(9,10-dihydro-5,8-dihydroxy-9,10-dioxo-1,4-anthracenediyl)bis(imino-2,1-ethanediyl)]bis[N-[(1,1-dimethylethoxy)carbonyl]-L-alanyl-L-alanyl-L-argininamide] dihydrobromide was slurried in 2.9 ml of anisole for 10 minutes in an ice bath. A 5.8 ml portion of trifluoroacetic acid was added, the mixture was stirred for 75 minutes, then quenched in 500 ml of ether. The mixture was filtered and dried in vacuo, giving 254 mg of the desired product, Rf 0.65 (system A).

Example 44

**N,N'-[(9,10-Dihydro-5,8-dihydroxy-9,10-dioxo-1,4-anthracenediyl)bis(imino-2,1-ethanediyl)]bis[N-[(phenylmethoxy)carbonyl]-L-arginyl-L-seryl-L-alaninamide] dihydrobromide**

A 2.0 g portion of carbobenzyloxy-L-arginine hydrobromide and 796 mg of hydroxybenzotriazole monohydrate were dissolved in 20 ml of dimethylformamide and then chilled to -10°C for 15 minutes. A 1.071 g portion of dicyclohexylcarbodiimide was dissolved in 10 ml of dimethylformamide and added to the above solution. This mixture was stirred for one hour at 0°C, then for one hour at room temperature and then filtered. The filtrate was added to a solution of 1.80 g of N,N'-[(9,10-dihydro-5,8-dihydroxy-9,10-dioxo-1,4-anthracenediyl)bis(imino-2,1-ethanediyl)]bis[L-seryl-L-alaninamide] bis(trifluoroacetate) and 1.4 ml of diisopropylethylamine in dimethylformamide. This mixture was stirred for 5 hours, then evaporated and the residue triturated with ethyl acetate, giving 3.94 g of crude product. Trituration with water and subsequent drying gave a purer product of Rf 0.8 in a tetrahydrofuran:acetic acid:water 13:2:1 tlc system, mp 150°C (dec.).

Example 45

**N,N'-[(9,10-Dihydro-5,8-dihydroxy-9,10-dioxo-1,4-anthracenediyl)bis(imino-2,1-ethanediyl)]bis[L-arginyl-L-seryl-L-alaninamide], tetrahydrobromide**

A 400 mg portion of the compound of Example 44 was added to 40 ml of water, stirred for 15 minutes and the solid recovered (230 mg). A 200 mg portion was added to 14 ml of hydrogen bromide saturated glacial acetic acid, stirred for one hour, quenched in ether and the solid collected and dried, giving 165 mg of the desired product, Rf 0.4 (system A).

Example 46

**N,N'-[(9,10-Dihydro-5,8-dihydroxy-9,10-dioxo-1,4-anthracenediyl)bis(imino-2,1-ethanediyl)]bis[N-[(phenylmethoxy)carbonyl]-L-seryl-L-alanyl-L-leucyl-L-leucinamide]**

A solution of 1.04 g of carbobenzyloxy-L-seryl-L-alanyl-L-leucyl-L-leucine and 230 mg of N-hydroxysuccinimide in 25 ml of dimethylformamide was cooled in an ice bath and 412 mg of dicyclohexylcarbodiimide was added. The mixture was stirred at 5°C for 16 hours and then filtered. A 320 mg portion of 1,4-bis[(2-aminoethyl)amino]-5,8-dihydroxyanthraquinone was added to the filtrate, then stirred at room temperature for 42 hours and centrifuged. The supernatant was decanted and the residue slurried with ether, giving 602 mg of the desired product, mp 193°C.

Example 47

**N,N'-[(9,10-Dihydro-5,8-dihydroxy-9,10-dioxo-1,4-anthracenediyl)bis(imino-2,1-ethanediyl)]bis-[L-seryl-L-alanyl-L-leucyl-L-leucinamide], dihydrobromide**

A 500 mg portion of N,N'-[(9,10-dihydro-5,8-dihydroxy-9,10-dioxo-1,4-anthracenediyl)bis(imino-2,1-ethanediyl)]-bis[N-[(phenylmethoxy)carbonyl]-L-seryl-L-alanyl-L-leucyl-L-leucinamide was dissolved in 15 ml of hydrogen bromide saturated glacial acetic acid, stirred for 4 hours and then diluted with 100 ml of ether. The solid was collected, washed with ether and dried in vacuo, giving 320 mg of the desired compound, HPLC 55:36:45:45 = 237 seconds.

Example 48

**N,N'-[(9,10-Dihydro-5,8-dihydroxy-9,10-dioxo-1,4-anthracenediyl)bis(imino-2,1-ethanediyl)]bis[N-[(9H-fluoren-9-ylmethoxy)carbonyl]-L-α-aspartyl-L-seryl-L-alanyl-L-leucyl-L-leucinamide] bis(1,1-dimethylethyl)ester**

Reaction of 246.9 mg of fluorenylmethyloxy carbonyl aspartic acid, β-tertiary butyl ester, 72.8 mg of N-methylmorpholine, 98 mg of isobutylchloroformate, and 82.9 mg of N-hydroxysuccinimide in 10 ml of ethyl acetate, as described in Example 10, gave the corresponding hydroxysuccinimide ester.

This ester in 4.5 ml of dimethylformamide was added to N,N'-[(9,10-dihydro-5,8-dihydroxy-9,10-dioxo-1,4-anthracenediyl)bis(imino-2,1-ethanediyl)]bis[L-seryl-L-alanyl-L-leucyl-L-leucinamide], dihydrobromide and 0.153 ml of diisopropylethylamine in 4.5 ml of dimethylformamide. This mixture was stirred for 16

hours, then filtered and the filtrate concentrated in vacuo. The residue was triturated with ether and the solid collected, washed with ether and dried in vacuo, giving 228 mg of the desired product, mp 188°C.

Example 49

**N,N'-[(9,10-Dihydro-5,8-dihydroxy-9,10-dioxo-1,4-anthracenediyl)bis(imino-2,1-ethanediyl)]bis[L-α-aspartyl-L-seryl-L-alanyl-L-leucyl-L-leucinamide], bis(1,1-dimethylethyl)ester**

To a 200 mg portion of N,N'-[(9,10-dihydro-5,8-dihydroxy-9,10-dioxo-1,4-anthracenediyl)bis(imino-2,1-ethanediyl)]bis[N-[(9H-fluoren-9-ylmethoxy)carbonyl)-L-α-aspartyl-L-seryl-L-alanyl-L-leucyl-L-leucin amide] bis(1,1-dimethylethyl)ester in 10 ml of dimethylformamide was added 10 ml of dimethylformamide saturated with dimethylamine. After standing 30 minutes the solution was concentrated in vacuo and the residue dried in vacuo, giving 152 mg of the desired compound, mp 205°C.

Example 50

**N,N'-[(9,10-Dihydro-5,8-dihydroxy-9,10-dioxo-1,4-anthracenediyl)bis(imino-2,1-ethanediyl)]bis-[N-[(9H-fluoren-9-ylmethoxy)carbonyl-L-α-aspartyl-L-alaninamide], bis(1,1-dimethylethyl)ester**

To a solution of fluorenylmethyloxycarbonyl aspartic acid, β-tertiary butyl ester, N-hydroxysuccinimide ester (prepared from 1.85 g of fluoroenylmethyloxycarbonyl aspartic acid, β-tertiary butyl ester, 564 mg of N-methylmorpholine, 738 mg of isobutylchloroformate and N-hydroxysuccinimide) in 50 ml of dimethylformamide, was added 1.03 g of N,N'-[(9,10-dihydro-5,8-dihydroxy-9,10-dioxo-1,4-anthracenediyl)bis(imino-2,1-ethanediyl)]bis[L-alaninamide] dihydrochloride and 1.16 g of diisopropylethylamine. The reaction mixture was stirred 72 hours, then filtered, the filtrate concentrated to a small volume in vacuo, then diluted with 150 ml of ether. The solid was collected, dried in vacuo, slurried in 50 ml of water and the solid collected and dried in vacuo, giving 1.2 g of the desired product, mp 190°C.

Example 51

**N,N'-[(9,10-Dihydro-5,8-dihydroxy-9,10-dioxo-1,4-anthracenediyl)bis(imino-2,1-ethanediyl)]bis[L-α-aspartyl-L-alaninamide], bis(1,1-dimethylethyl)ester**

To 1.1 g of N,N'-[(9,10-dihydro-5,8-dihydroxy--9,10-dioxo-1,4-anthracenediyl)bis(imino-2,1-ethanediyl)]-bis[N-[(9H-fluoren-9-ylmethoxy)carbonyl]-L-α-aspartyl-L-alanamide] bis(1,1-dimethylethyl)ester in 25 ml of dimethylformamide was added 25 ml of dimethylformamide saturated with dimethylamine. After standing 30

minutes the mixture was concentrated to a small volume and then diluted with 100 ml of ether. The solid was collected, washed with ether and dried in vacuo, giving 908 mg of the desired product, mp 200°C.

Example 52

**N,N'-[(9,10-Dihydro-5,8-dihydroxy-9,10-dioxo-1,4-anthracenediyl)bis(imino-2,1-ethanediyl)]bis[N-[(9H-fluoren-9-ylmethoxy)carbonyl]glycylglycyl-L-α-aspartyl-L-alaninamide], bis(1,1-dimethylethyl)ester**

To a solution of fluorenylmethyloxycarbonylglycylglycine hydroxysuccinimide ester (prepared from 443 mg of fluoroenylmethyloxycarbonylglycylglycine and 159 mg of N-hydroxysuccinimide and dicyclohexylcarbodiimide) in 75 ml of dimethylformamide was added 598.6 mg of N,N'-[(9,10-dihydro-5,8-dihydroxy-9,10-dioxo-1,4-anthracenediyl)bis(imino-2,1-ethanediyl)]bis[L-α-aspartyl-L-alaninamide] bis(1,1-dimethylethyl)ester and 248 mg of diisopropylethylamine. The mixture was stirred overnight, then filtered and the filtrate concentrated to a small volume. This was diluted with ether, the solid collected, washed with ether and dried in vacuo, giving 915 mg of the desired product, mp 175°C.

Example 53

**N,N'-[(9,10-Dihydro-5,8-dihydroxy-9,10-dioxo-1,4-anthracenediyl)bis(imino-2,1-ethanediyl)]bis[glycylglycyl-L-α-aspartyl-L-alaninamide], bis(1,1-dimethylethyl)ester**

To 800 mg of N,N'-[(9,10-dihydro-5,8-dihydroxy-9,10-dioxo-1,4-anthracenediyl)bis(imino-2,1-ethanediyl)-]bis[N-[(9H-fluoren-9-ylmethoxy)carbonyl] glycylglycyl-L-α-aspartyl-L-alaninamide], bis(1,1-dimethylethyl)-ester in 25 ml of dimethylformamide was added 25 ml of dimethylformamide saturated with dimethylamine. After 30 minutes the mixture was concentrated to a small volume, then diluted with 150 ml of ether, the solid collected, washed with ether and dried in vacuo, giving 614 mg of the desired product, mp 180°C.

Example 54

**N,N'-[(9,10-Dihydro-5,8-dihydroxy-9,10-dioxo-1,4-anthracenediyl)bis(imino-2,1-ethanediyl)]bis[N-[(9H-fluoren-9-ylmethoxy)carbonyl]-L-α-aspartyl-glycyl-L-alanyl-L-prolinamide], bis(1,1-dimethylethyl)ester**

A solution of fluorenylmethyloxycarbonyl-L-α-aspartyl-(γ-O-tertiary-butyl ester)-glycyl-L-alanyl-L-proline

and N-hydroxysuccinimide in 30 ml of dimethylformamide was cooled in an ice bath and dicyclohexylcarbodiimide added. After 16 hours at 5°C the reaction mixture was filtered and 142 mg of 1,4-bis[(2-aminoethyl)amino]-5,8-dihydroxyanthraquinone was added. After 60 hours at room temperature the mixture was centrifuged, the supernatant filtered and the filtrate concentrated in vacuo. The residue was slurried with ether, the solid collected, washed with ether and dried in vacuo, giving 763 mg of the desired product, mp 160°C.

Example 55

**N,N'-[(9,10-Dihydro-5,8-dihydroxy-9,10-dioxo-1,4-anthracenediyl)bis(imino-2,1-ethanediyl)]bis-[L-α-aspartyl-glycyl-L-alanyl-L-prolinamide], bis-(1,1-dimethylethyl)ester**

To a solution of 700 mg of N,N'-[(9,10-dihydro-5,8-dihydroxy-9,10-dioxo-1,4-anthracendiyl)bis(imino-2,1-ethandiyl)]bis[N-[(9H-fluoren-9-ylmethoxy)carbonyl]-L-α-aspartyl-glycyl-L-alanyl-L-prolinamide], bis(1,1-dimethylethyl)ester in 25 ml of dimethylformamide was added 25 ml of dimethylformamide saturated with dimethylamine. After one hour the mixture was concentrated in vacuo, the residue slurried with ether, the solid collected, washed with ether and dried in vacuo, giving 501 mg of the desired compound, mp 145°C.

Example 56

**N,N'-[(9,10-Dihydro-5,8-dihydroxy-9,10-dioxo-1,4-anthracenediyl)bis(imino-2,1-ethanediyl)]bis[N-[(phenylmethoxy)carbonyl]-L-phenylalanyl-L-leucinamide]**

A mixture of 2.73 g of carbobenzyloxy-L-phenylalanyl hydroxysuccinimide ester, 2.6 ml of triethylamine and 2.43 g of [S-(R*,R*)]-N,N'-[(9,10-dihydro-5,8-dihydroxy-9,10-dioxo-1,4-anthracenediyl)bis(imino-2,1-ethanediyl)]bis[2-amino-4-methylpentanamide] bis(trifluoroacetate) (prepared as described in Example 5) in 100 ml of dimethylformamide was reacted as described in Example 14, giving 3.215 g of the desired product, mp 240°C (dec.).

Example 57

**N,N'-[(9,10-Dihydro-5,8-dihydroxy-9,10-dioxo-1,4-anthracenediyl)bis(imino-2,1-ethanediyl)]bis[L-phenylalanyl-L-leucinamide], dihydrobromide**

A 2.75 g portion of N,N'-[(9,10-dihydro-5,8-dihydroxy-9,10-dioxo-1,4-anthracenediyl)bis(imino-2,1-ethanediyl)]bis[N-[(phenylmethoxy)carbonyl]-L-phenylalanyl-L-leucinamide] was reacted by a modification of the procedure of Example 8, by dissolving in 75 ml of glacial acetic acid previously saturated for 5 minutes with anhydrous hydrogen bromide. The mixture was reacted for 2.5 hours, then more hydrogen bromide was bubbled into the solution for 15 minutes and after another 2 hours the solid was collected, giving 2.7 g

of the desired product, mp 180°C (dec.).

Example 58

**N,N'-[(9,10-Dihydro-5,8-dihydroxy-9,10-dioxo-1,4-anthracenediyl)bis(imino-2,1-ethanediyl)]bis[N-[(phenylmethoxy)carbonyl]-L-phenylalanyl-L-phenylalanyl-L-leucinamide]**

Reaction of 1.822 g of carbobenzyloxy-L-phenylalanyl hydroxysuccinimide ester with 2.076 g of N,N'-[-(9,10-dihydro-5,8-dihydroxy-9,10-dioxo-1,4-anthracenediyl)bis(imino-2,1-ethanediyl)]bis[L-phenylalanyl-L-leucinamide], dihydrobromide and 1.73 ml of triethylamine, in the manner of Example 14, gave 2.3 g of the desired product, mp 250°C (dec.).

Example 59

**N,N'-[(9,10-Dihydro-5,8-dihydroxy-9,10-dioxo-1,4-anthracenediyl)bis(imino-2,1-ethanediyl)]bis[L-phenylalanyl-L-phenylalanyl-L-leucinamide], dihydrobromide**

Reaction of 2.0 g of N,N'-[(9,10-dihydro-5,8-dihydroxy-9,10-dioxo-1,4-anthracendiyl)bis(imino-2,1-ethanediyl)]bis[N-[(phenylmethoxy)carbonyl]-L-phenylalanyl-L-phenylalanyl-L-leucinamide] with 60 ml of hydrogen bromide-saturated glacial acetic acid for 2 hours following the general method of Example 8, gave 1.65 g of the desired product, mp 197°C.

Example 60

**N,N'-[(9,10-Dihydro-5,8-dihydroxy-9,10-dioxo-1,4-anthracenediyl)bis(imino-2,1-ethanediyl)]bis[N-[(1,1-dimethylethoxy)carbonyl]glycyl-L-phenylalanyl-L-phenylalanyl-L-leucinamide]**

Following the method of Example 14, 1.34 g of N,N'-[(9,10-dihydro-5,8-dihydroxy-9,10-dioxo-1,4-anthracenediyl)bis(imino-2,1-ethanediyl)]bis[L-phenylalanyl--L-phenylalanyl-L-leucinamide], dihydrobromide, 0.68 g of t-butyloxycarbonyl-glycyl hydroxysuccinimide ester and 0.86 ml of triethylamine in 100 ml of dimethylformamide were reacted, giving 1.16 g of the desired product, mp >260°C.

Example 61

**N,N'-[(9,10-Dihydro-5,8-dihydroxy-9,10-dioxo-1,4-anthracenediyl)bis(imino-2,1-ethanediyl)]bis[glycyl-L-phenylalanyl-L-phenylalanyl-L-leucinamide], bis(trifluoroacetate)**

Using a slight modification of the procedure of Example 20, 0.66 g of N,N'-[(9,10-dihydro-5,8-dihydroxy-9,10-dioxo-1,4-anthracenediyl)bis(imino-2,1-ethanediyl)]bis[N-[(1,1-dimethylethoxy)carbonyl]glycyl-L-phenylalanyl-L-phenylalanyl-L-leucinamide] in 6.6 ml of anisole and 13 ml of trifluoroacetic acid was reacted for 5 minutes at 5°C and then 3 hours at room temperature, giving 0.58 g of the desired product, mp 255°C.

Example 62

**N,N'-[(9,10-Dihydro-5,8-dihydroxy-9,10-dioxo-1,4-anthracenediyl)bis(imino-2,1-ethanediyl)]bis[N-[(9H-fluoren-9-ylmethoxy)carbonyl]-L-α-aspartyl-L-leucyl-L-alanyl-L-leucinamide], bis(1,1-dimethyethyl)ester**

A 1.21 g portion of fluorenylmethyloxycarbonyl aspartic acid, β-tertiary butyl ester was converted to the corresponding hydroxysuccinimide ester, according to the procedure of Example 10. This ester was reacted with N,N'-[(9,10-dihydro-5,8-dihydroxy-9,10-dioxo-1,4-anthracenediyl)bis(imino-2,1-ethanediyl)]bis[L-leucyl-L-alanyl-L-leucinamide] dihydrobromide in 75 ml of dimethylformamide, by the procedure of Example 14 in the presence of 0.73 ml of diisopropylethylamine, giving 1.64 g of the desired product, mp 240°C (dec.).

Example 63

**N,N'-[(9,10-Dihydro-5,8-dihydroxy-9,10-dioxo-1,4-anthracenediyl)bis(imino-2,1-ethanediyl)]bis[L-α-aspartyl-L-leucyl-L-alanyl-L-leucinamide], bis(1,1-dimethylethyl)ester**

A solution of 1.3 g of N,N'-[(9,10-dihydro-5,8-dihydroxy-9,10-dioxo-1,4-anthracenediyl)bis(imino-2,1-ethanediyl)]bis[N-[9H-fluoren-9-ylmethoxy)carbonyl]-L-α-aspartyl-L-leucyl-L-alanyl-L-leucinamide], bis(1,1-dimethylethyl)ester in 50 ml of dimethylformamide was treated for 10 minutes with dimethylamine, as described in Example 11, giving 0.88 g of the desired product, mp 251°C (dec.).

Example 64

**N,N'-[(9,10-Dihydro-5,8-dihydroxy-9,10-dioxo-1,4-anthracenediyl)bis(imino-2,1-ethanediyl)]bis[N-[(9H-fluoren-9-ylmethoxy)carbonyl]glycyl-L-α-aspartyl-L-alanyl-L-leucinamide], bis(1,1-dimethylethyl)ester**

A solution of fluorenylmethyloxycarbonylglycine, hydroxysuccinimide ester was prepared by reaction of 0.3 g of fluorenylmethyloxycarbonylglycine, 0.13 g of N-hydroxysuccinimide and 0.23 g of dicyclohexylcarbodiimide in 5 ml of tetrahydrofuran for 1 hour at 5°C, then 18 hours at 1O°C. Filtration of the dicyclohexyl urea precipitate and evaporation of the filtrate gave the hydroxysuccinimide ester. This ester was dissolved in dimethylformamide and reacted with 0.36 g of N,N'-[(9,10-dihydro-5,8-dihydroxy-9,10-dioxo-1,4-anthracenediyl)bis(imino-2,1-ethanediyl)]bis[N-[(9H-fluoren-9-ylmethoxy)carbonyl]-L-α-aspartyl-L-alanyl-L-leucinamide] bis(1,1-dimethylethyl)ester by the procedure of Example 10, giving 0.4 g of the desired product, mp 225°C (dec.).

Example 65

**N,N'-[(9,10-Dihydro-5,8-dihydroxy-9,10-dioxo-1,4-anthracenediyl)bis[imino-2,1-ethanediylimino-[1-[2-(1,1-dimethylethoxy)-2-oxoethyl]-2-oxo-2,1-ethanediyl]]]bis[N-[(9H-fluoren-9-ylmethoxy)-carbonyl]-L-leucyl-L-alanyl-L-leucinamide]**

A solution of fluorenylmethyloxycarbonyl-L-leucyl-L-alanyl-L-leucine hydroxysuccinimide ester was prepared from 1.08 g of the corresponding acid and 2.2 mmole each of N-hydroxysuccinimide and dicyclohexylcarbodiimide in 15 ml of tetrahydrofuran as described in Example 64. Reaction with 0.48 g of [S-(R*,R*)]-4,4'[(9,10-dihydro-5,8-dihydroxy-9,10-dioxo-1,4-anthracenediyl)bis(imino-2,1-ethanediylimino)]bis[3-amino-4-oxobutanoic acid] bis(1,1-dimethylethyl)ester, following the procedure of Example 10 gave 1.47 g of the desired product in two crops, mp 200°C (dec.).

Example 66

**N,N'-[(9,10-Dihydro-5,8-dihydroxy-9,10-dioxo-1,4-anthracenediyl)bis(imino-2,1-ethanediyl)]bis[N-[(9H-fluoren-9-ylmethoxy)carbonyl]glycylglycyl-L-α-aspartyl-L-alanyl-L-leucinamide] bis(1,1-dimethylethyl)ester**

A solution of fluorenylmethyloxycarbonyl-glycylglycine hydroxysuccinimide ester was prepared from 0.52 g of fluorenylmethyloxycarbonyl-glycylglycine, 0.19 g of N-hydroxysuccinimide and 0.33 g of dicyclohexylcarbodiimide in 30 ml of tetrahydrofuran, as described in Example 64. This was reacted with 0.53 g of N,N'-[(9,10-dihydro-5,8-dihydroxy-9,10-dioxo-1,4-anthracenediyl)bis(imino-2,1-ethanediyl)]bis[N-(9H-fluoren-9-ylmethoxy)carbonyl]-L-α-aspartyl-L-alanyl-L-leucinamide] bis(1,1-dimethylethyl)ester following

the procedure of Example 10, giving 0.66 g of the desired product, mp 220°C (dec.).

Example 67

**N,N'-[(9,10-Dihydro-5,8-dihydroxy-9,10-dioxo-1,4-anthracenediyl)bis(imino-2,1-ethanediylimino)]bis[[1-[2-(1,1-dimethylethoxy)-2-oxoethyl]-2-oxoethyl]-2-oxo-2,1-ethanediyl]bis[L-leucyl-L-alanyl-L-leucinamide]**

A solution of 0.4 g of N,N'-[(9,10-dihydro-5,8-dihydroxy-9,10-dioxo-1,4-anthrancenediyl)bis[imino-2,1-ethanediylimino-[1-[2-(1,1-dimethylethoxy)-2-oxoethyl]-2-oxo-2,1-ethanediyl]]]bis[N-[(9H-fluoren-9-ylmethoxy)carbonyl]-L-leucyl-L-alanyl-L-leucinamide] was treated with dimethylamine in 4 ml of dimethylformamide, as described in Example 11, giving 0.26 g of the desired product, mp 205°C (dec.).

Example 68

**N,N'-[(9,10-Dihydro-5,8-dihydroxy-9,10-dioxo-1,4-anthracenediyl)bis(imino-2,1-ethanediyl)]bis[glycylglycyl-L-α-aspartyl-L-alanyl-L-leucinamide], bis(1,1-dimethylethyl)ester**

A solution of 0.4 g of N,N'-[(9,10-dihydro-5,8-dihydroxy-9,10-dioxo-1,4-anthracenediyl)bis(imino-2,1-ethanediyl)]bis[N-[(9H-fluoren-9-ylmethoxy)carbonyl]glycylglycyl-L-α-aspartyl-L-alanyl-L-leucinamide], bis-(1,1-dimethylethyl) ester was treated with dimethylamine in 8 ml of dimethylformamide as described in Example 11, giving 0.3 g of the desired product, mp 228°C (dec.).

Example 69

**N,N'-[9,10-Dihydro-5,8-dihydroxy-9,10-dioxo-1,4-anthracenediyl)bis(imino-2,1-ethanediyl)]bis[glycyl-L-α-aspartyl-L-alanyl-L-leucinamide], bis(1,1-dimethylethyl)ester**

A solution of 0.3 g of N,N'-[(9,10-dihydro-5,8-dihydroxy-9,10-dioxo-1,4-anthracenediyl)bis(imino-2,1-ethanediyl)]bis[N-(9H-fluoren-9-ylmethoxy)carbonyl]glycyl-L-α-aspartyl-L-alanyl-L-leucinamide, bis(1,1-dimethylethyl)ester in 6 ml of dimethylformamide was treated with dimethylamine, as described in Example 11, giving 0.2 g of the desired product, mp 255°C (dec.).

## Synthesis of N-succinyl Compounds from peptide derivatives of 1,4-bis[(2-aminoethyl)-amino]-5,8-dihydroxyanthraquinones

Example 70

### N,N'-[(9,10-Dihydro-5,8-dihydroxy-9,10-dioxo-1,4-anthracenediyl)bis(imino-2,1-ethanediyl)]-bis[N-(3-carboxy-1-oxopropyl)-L-α-aspartyl-L-alanyl-L-alaninamide] bis(1,1-dimethylethyl)-ester

A mixture of 100 mg of N,N'-[(9,10-dihydro-5,8-dihydroxy-9,10-dioxo-1,4-anthracenediyl)bis(imino-2,1-ethanediyl)]bis[L-α-aspartyl-L-alanyl-L-alaninamide] bis(1,1-dimethylethyl)ester, 22.0 mg of succinic anhydride and 29.0 mg of triethylamine in 4.0 ml of N,N-dimethylformamide was stirred at room temperature for 20 hours. Then the reaction mixture was poured into 100 ml of diethyl ether. The product was collected by filtration and dried in vacuo for several hours. The dried material was triturated with cold water, then acidified carefully to pH 2.0 with 5% phosphoric acid. The solid was collected by filtration, washed with cold water and dried in vacuo to give 89 mg (75% yield) of the desired product.

Additional N-succinyl compounds which were prepared from their peptide derivative precursors in a manner similar to that described in Example 70 are listed in Table III as Examples 71-94.

## Table III

**N-SUCCINYL COMPOUNDS PREPARED FROM PEPTIDE DERIVATIVES OF 1,4-BIS[(2-AMINOETHYL)AMINO]-5,8-DIHYDROXYANTHRAQUINONE**

| Example | Precursor | Compound | MP°C |
|---|---|---|---|
| 71 | Ex. 6 | [R-(R*,R*)]-4,4'[(9,10-Dihydro-5,8-dihydroxy-9,10-dioxo-1,4-anthracenediyl)bis[imino-2,1-ethanediylimino(1-methyl-2-oxo-2,1-ethanediylimino]]bis[4-oxobutanoic acid] | 230(dec) |
| 72 | Ex. 4 | [S-(R*,R*)]-4,4'-[(9,10-Dihydro-5,8-dihydroxy-9,10-dioxo-1,4-anthracenediyl)bis[imino-2,1-ethanediylimino(1-methyl-2-oxo-2,1-ethanediyl)imino]]bis[4-oxobutanoic acid] | 240(dec) |
| 73 | Ex. 5 | 4,4'-[(9,10-Dihydro-5,8-dihydroxy-9,10-dioxo-1,4-anthracenediyl)bis[imino-2,1-ethanediylimino[1-(2-methylpropyl)-2-oxo-2,1-ethanediyl]imino]]bis[4-oxobutanoic acid] | |
| 74 | Ex. 13 | N,N'-[(9,10-Dihydro-5,8-dihydroxy-9,10-dioxo-1,4-anthrancenediyl)-bis(imino-2,1-ethanediyl)]bis[(3-carboxy-1-oxopropyl)-L-alanyl-L-leucinamide] | |
| 75 | Ex. 28 | N,N'-[(9,10-Dihydro-5,8-dihydroxy-9,10-dioxo-1,4-anthracenediyl)bis(imino-2,1-ethanediyl)]bis[(3-carboxy-1-oxopropyl)-L-alanyl-L-alaninamide] | |

Table III (continued)

| Example | Precursor | Compound | MP°C |
|---|---|---|---|
| 76 | Ex. 18 | N,N'-[(9,10-Dihydro-5,8-dihydroxy-9,10-dioxo-1,4-anthracenediyl)bis(imino-2,1-ethanediyl)]bis[N-(3-carboxy-1-oxopropyl)-glycylglycinamide] | |
| 77 | Ex. 22 | N,N'-[(9,10-Dihydro-5,8-dihydroxy-9,10-dioxo-1,4-anthracenediyl)bis(imino-2,1-ethanediyl)]bis[N-(3-carboxy-1-oxopropyl)-L-leucyl-L-alaninamide] | |
| 78 | Ex. 15 | N,N'-[(9,10-Dihydro-5,8-dihydroxy-9,10-dioxo-1,4-anthracenediyl)bis(imino-2,1-ethanediyl)]bis[N-(3-carboxy-1-oxopropyl-L-leucyl-L-alanyl-L-leucinamide] | >200 |
| 79 | Ex. 20 | N,N'-[(9,10-Dihydro-5,8-dihydroxy-9,10-dioxo-1,4-anthracenediyl)bis(imino-2,1-ethanediyl)]bis[N-(3-carboxy-1-oxopropyl)-glycylglycylglycinamide] | 150(dec) |
| 80 | Ex. 26 | N,N'-[(9,10-Dihydro-5,8-dihydroxy-9,10-dioxo-1,4-anthracenediyl)bis(imino-2,1-ethanediyl)]bis[N-(3-carboxy-1-oxopropyl)-glycylglycyl-L-leucinamide] | 195(dec) |
| 81 | Ex. 24 | N,N'-[(9,10-Dihydro-5,8-dihydroxy-9,10-dioxo-1,4-anthracenediyl)bis(imino-2,1-ethanediyl)]bis[N-(3-carboxy-1-oxopropyl)-L-alanyl-L-alanyl-L-leucinamide] | >200(dec) |

## Table III (continued)

| Example | Precursor | Compound | MP°C |
|---|---|---|---|
| 82 | Ex. 30 | N,N'-[(9,10-Dihydro-5,8-dihydroxy-9,10-dioxo-1,4-anthracenediyl)bis(imino-2,1-ethanediyl)]bis[N-(3-carboxy-1-oxopropyl)-L-alanyl-L-alanyl-L-alaninamide] | >200(dec) |
| 83 | Ex. 33 | N,N'-[(9,10-Dihydro-5,8-dihydroxy-9,10-dioxo-1,4-anthracenediyl)bis(imino-2,1-ethanediyl)]bis[N-(3-carboxy-1-oxopropyl)-L--alpha-aspartyl-L-seryl-L-alaninamide] bis(1,1-dimethylethyl)ester | |
| 84 | Ex. 37 | N,N'-[(9,10-Dihydro-5,8-dihydroxy-9,10-dioxo-1,4-anthracenediyl)bis(imino-2,1-ethanediyl)]bis[N-(3-carboxy-1-oxopropyl-L-alpha-aspartyl-L-alanyl-L-leucinamide] bis(1,1-dimethylethyl)-ester | |
| 85 | Ex. 45 | N,N'-[(9,10-Dihydro-5,8-dihydroxy-9,10-dioxo-1,4-anthracenediyl)bis(imino-2,1-ethanediyl)]bis[N-(3-carboxy-1-oxopropyl)-L-arginyl-L-seryl-L-alaninamide], dihydrobromide | |
| 86 | Ex. 43 | N,N'-[(9,10-Dihydro-5,8-dihydroxy-9,10-dioxo-1,4-anthracenediyl)bis(imino-2,1-ethanediyl)]bis[N-(3-carboxy-1-oxopropyl)-L-alanyl-L-alanyl-L-argininamide], dihydrobromide | |
| 87 | Ex. 61 | N,N'-[(9,10-Dihydro-5,8-dihydroxy-9,10-dioxo-1,4-anthracenediyl)bis(imino-2,1-ethanediyl)]bis[N-(3-carboxy-1-oxopropyl)-glycyl-L-phenylalanyl-L-phenylalanyl-L-leucinamide] | |

Table III (continued)

| Example | Precursor | Compound | MP°C |
|---|---|---|---|
| 88 | Ex. 63 | N,N'-[(9,10-Dihydro-5,8-dihydroxy-9,10-dioxo-1,4-anthracenediyl)bis(imino-2,1-ethanediyl)]bis[N-(3-carboxy-1-oxopropyl)-glycyl-L-alpha-aspartyl-L-leucyl-L-alanyl-L-leucinamide] bis(1,1-dimethylethyl)ester | |
| 89 | Ex. 67 | N,N'-[(9,10-Dihydro-5,8-dihydroxy-9,10-dioxo-1,4-anthracenediyl)bis[imino-2,1-ethanediylimino[1-[2-(1,1-dimethylethoxy)-2-oxoethyl]-2-oxo-2,1-ethanediyl]]]bis[N-(3-carboxy-1-oxopropyl)-L-leucyl-L-alanyl-L-leucinamide] | |
| 90 | Ex. 68 | N,N'-[(9,10-Dihydro-5,8-dihydroxy-9,10-dioxo-1,4-anthracenediyl)bis(imino-2,1-ethanediyl)]bis[N-(3-carboxy-1-oxopropyl)-glycylglycyl-L-alpha-aspartyl-L-alanyl-L-leucinamide] bis(1,1-dimethylethyl)ester | |
| 91 | Ex. 69 | N,N'-[(9,10-Dihydro-5,8-dihydroxy-9,10-dioxo-1,4-anthracenediyl)bis(imino-2,1-ethanediyl)]bis[N-(3-carboxy-1-oxopropyl)-glycyl-L-alpha-aspartyl-L-alanyl-L-leucinamide] bis(1,1-dimethylethyl)ester | |
| 92 | Ex. 43 | N,N'-[(9,10-Dihydro-5,8-dihydroxy-9,10-dioxo-1,4-anthracenediyl)bis(imino-2,1-ethanediyl)]bis[N-(3-carboxy-1-oxopropyl)-L-alpha-aspartyl-L-seryl-L-leucyl-L-leucinamide] bis(1,1-dimethylethyl)ester | |

**Table III (continued)**

| Example | Precursor | Compound | MP°C |
|---|---|---|---|
| 93 | Ex. 53 | N,N'-[(9,10-Dihydro-5,8-dihydroxy-9,10-dioxo-1,4-anthracenediyl)bis(imino-2,1-ethanediyl)]bis[N-(3-carboxy-1-oxopropyl)-glycylglycyl-L-alpha-aspartyl-L-alaninamide] bis(1,1-dimethylethyl)ester | |
| 94 | Ex. 55 | N,N'-[(9,10-Dihydro-5,8-dihydroxy-9,10-dioxo-1,4-anthracenediyl)bis(imino-2,1-ethanediyl)]bis[N-(3-carboxy-1-oxopropyl)-L-alpha-aspartyl-glycyl-L-alanyl-L-prolinamide] bis(1,1-dimethylethyl)ester | |

## Synthesis of N-(Succinyl)-Aspartic Acid Peptide Derivatives of 1,4-Bis[(2-aminoethyl)amino]-5,8-dihyroxyanthraquinone from their corresponding Aspartic-β-O-tertiary-butyl esters

Example 95

### N,N'-[9,10-Dihydro-5,8-dihydroxy-9,10-dioxo-1,4-anthracenediyl)bis(imino-2,1-ethanediyl)]bis[N-(3-carboxy-1-oxopropyl)glycyl-L-α-aspartyl-L-alanyl-L-leucinamide

To a stirred slurry of 16 mg of N,N'-[(9,10-dihydro-5,8-dihydroxy-9,10-dioxo-1,4-anthracenediyl)bis-(imino-2,1-ethanediyl)]bis[N-(3-carboxy-1-oxopropyl) glycyl-L-α-aspartyl-L-alanyl-L-leucinamide] bis(1,1-dimethylethyl)ester in 0.15 ml of anisole, cooled in an ice bath was added 0.3 ml of trifluoroacetic acid. After 15 minutes in the cold the mixture was stirred at room temperature for one hour and 15 minutes, then added to an excess of diethyl ether. The solid was collected and dried to give 11 mg of the desired product.

Additional N-(succinyl)-aspartic acid-containing peptide derivatives which were prepared from the corresponding aspartic-β-O-tertiary-butyl ester precursors in a manner similar to that described in Example 95 are listed in Table IV as Examples 96-103.

## Table IV

**N-(SUCCINYL)-ASPARTIC ACID PEPTIDE DERIVATIVES OF 1,4-BIS[(2-AMINOETHYL)-AMINO]-5,8-DIHYDROXYANTHRAQUINONE PREPARED FROM CORRESPONDING ASPARTIC--O-TERTIARY-BUTYL ESTERS**

| Example | Precursor | Compound |
|---|---|---|
| 96 | Ex. 83 | N,N'-[(9,10-Dihydro-5,8-dihydroxy-9,10-dioxo-1,4-anthracenediyl)bis-(imino-2,1-ethanediyl)]bis[N-(3-carboxy-1-oxopropyl)-L-alpha-aspartyl-L-seryl-L-alaninamide] |
| 97 | Ex. 70 | N,N'-[(9,10-Dihydro-5,8-dihydroxy-9,10-dioxo-1,4-anthracenediyl)-bis-(imino-2,1-ethanediyl)]bis[N-(3-carboxy-1-oxopropyl)-L-alpha-aspartyl-L-alanyl-L-alaninamide] |
| 98 | Ex. 84 | N,N'-[(9,10-Dihydro-5,8-dihydroxy-9,10-dioxo-1,4-anthracenediyl)bis-(imino-2,1-ethanediyl)]bis[N-(3-carboxy-1-oxopropy)-L-alpha-aspartyl-L-alanyl-L-leucinamide] |
| 99 | Ex. 88 | N,N'-[(9,10-Dihydro-5,8-dihydroxy-9,10-dioxo-1,4-anthracenediyl)bis-(imino-2,1-ethanediyl)]bis[N-(3-carboxy-1-oxopropyl)-L-alpha-aspartyl-L-leucyl-L-alanyl-L-leucinamide] |
| 100 | Ex. 89 | N,N'-[(9,10-Dihydro-5,8-dihydroxy-9,10-dioxo-1,4-anthracenediyl)bis-(imino-2,1-ethanediylimino[1-carboxymethyl)-2-oxo-2,1-ethanediyl]]]bis-[N-(3-carboxy-1-oxopropyl)-L-leucyl-L-alanyl-L-leucinamide] |

Table IV (continued)

| Example | Precursor | Compound |
|---|---|---|
| 101 | Ex. 90 | N,N'-[(9,10-Dihydro-5,8-dihydroxy-9,10-dioxo-1,4-anthracenediyl)bis-(imino-2,1-ethanediyl)]bis[N-(3-carboxy-1-oxopropyl)glycylglycyl-L-alpha-aspartyl-L-alanyl-L-leucinamide] |
| 102 | Ex. 92 | N,N'-[(9,10-Dihydro-5,8-dihydroxy-9,10-dioxo-1,4-anthracenediyl)-bis(imino-2,1-ethanediyl)]bis[N-(3-carboxy-1-oxopropyl)-L-alpha-aspartyl-L-seryl-L-alanyl-L-leucyl-L-leucinamide] |
| 103 | Ex. 93 | N,N'-[(9,10-Dihydro-5,8-dihydroxy-9,10-dioxo-1,4-anthracenediyl)bis-(imino-2,1-ethanediyl)]bis[N-(3-carboxy-1-oxopropyl)glycylglycyl-L-alpha-aspartyl-L-alaninamide] |

## Synthesis of N-Hydroxysuccinimide Ester Compounds from their Corresponding N-Succinic Acids

Example 104

### [S-(R*,R*)]-N,N'-[(9,10-Dihydro-5,8-dihydroxy-9,10-dioxo-1,4-anthracenediyl)bis[imino-2,1-ethanediylimino(1-methyl-2-oxo-2,1-ethanediyl)]]bis[4-[(2,5-dioxo-1-pyrrolidinyl)oxy]-4-oxobutanamide]

A solution of 45.0 mg of [S-(R*,R*)]-4,4'-[(9,10-dihydro-5,8-dihydroxy-9,10-dioxo-1,4-anthracenediyl)bis-[imino-2,1-ethanediylimino(1-methyl-2-oxo-2,1-ethanediyl)imino]]bis-[4-oxobutanoic acid], 16.0 mg of N-hydroxysuccinimide and 29.0 mg of dicyclohexylcarbodiimide in 4.0 ml of N,N-dimethylformamide was stirred for 16 hours. Then an additional 8.0 mg of N-hydroxysuccinimide and 15.0 mg dicyclohexycarbodiimide was added when tlc, chloroform:methanol 85:15, showed starting material present.

After stirring 24 hours longer the same quantities of reagents were again added. Finally after the third overnight stirring, tlc showed the reaction was complete. The mixture was filtered using dry equipment and the filtrate was evaporated to dryness. The residue was triturated with ethyl acetate and the crude product was collected by filtration and dried. The product was purified by stirring in isopropyl alcohol for 2 hours to give 27 mg of the desired product in 47% yield, mp 180°C (dec.).

Other hydroxysuccinimide ester compounds which were prepared from the corresponding N-succinic acids in a similar manner to that described in Example 104 are listed in Table V as Examples 105-120.

## Table V

**N-HYDROXYSUCCINIMIDE ESTER COMPOUNDS PREPARED FROM THEIR CORRESPONDING SUCCINIC ACIDS**

| Example | Precursor | Compound | MP°C |
|---|---|---|---|
| 105 | Ex. 71 | [R-(R*,R*)]-N,N'-[(9,10-Dihydro-5,8-dihydroxy-9,10-dioxo-1,4-anthracenediyl)bis[imino-2,1-ethanediylimino(1-methyl-2-oxo-2,1-ethanediyl)]]bis[4-[(2,5-dioxo-1-pyrrolidinyl)oxy]-4-oxo-butanamide] | 180(dec) |
| 106 | Ex. 78 | N,N'-[(9,10-Dihydro-5,8-dihydroxy-9,10-dioxo-1,4-anthracenediyl)bis(imino-2,1-ethanediyl)]bis[N-[4-[(2,5-dioxo-1-pyrrolidinyl)oxy]-1,4-dioxobutyl]-L-leucyl-L-alanyl-L-leucinamide] | 220 |
| 107 | Ex. 79 | N,N'-[(9,10-Dihydro-5,8-dihydroxy-9,10-dioxo-1,4-anthracenediyl)bis(imino-2,1-ethanediyl)bis[N-[1,4-dioxo-4-[(2,5-dioxo-1-pyrrolidinyl)oxy]butyl]glycylglycylglycinamide] | 100(dec) |
| 108 | Ex. 80 | N,N'-[(9,10-Dihydro-5,8-dihydroxy-9,10-dioxo-1,4-anthracenediyl)bis(imino-2,1-ethanediyl)]bis[N-[1,4-dioxo-4-[2,5-dioxo-1-pyrrolidinyl)oxy]butyl]glycylglycyl-L-leucinamide] | 198(dec) |
| 109 | Ex. 81 | N,N'-[(9,10-Dihydro-5,8-dihydroxy-9,10-dioxo-1,4-anthracenediyl)bis(imino-2,1-ethanediyl)]bis[N-[1,4-dioxo-[(2,5-dioxo-1-pyrrolidinyl)oxy]butyl]-L-alanyl-L-alanyl-L-leucinamide] | >200(dec) |

**Table V (continued)**

| Example | Precursor | Compound | MP°C |
|---|---|---|---|
| 110 | Ex. 83 | N,N'-[(9,10-Dihydro-5,8-dihydroxy-9,10-dioxo-1,4-anthracenediyl)bis(imino-2,1-ethanediyl)]bis[N-[4-[(2,5-dioxo-1-pyrrolidinyl)oxy]-1,4-dioxobutyl]-L-alpha-aspartyl-L-seryl-L-alanamide] bis(1,1-dimethylethyl)ester | |
| 111 | Ex. 70 | N,N'-[(9,10-Dihydro-5,8-dihydroxy-9,10-dioxo-1,4-anthracenediyl)bis(imino-2,1-ethanediyl)]bis[N-[4-[(2,5-dioxo-1-pyrrolidinyl)oxy]-1,4-dioxobutyl]-L-alpha-aspartyl-L-alanyl-L-alaninamide] bis(1,1-dimethylethyl)ester | |
| 112 | Ex. 84 | N,N'-[(9,10-Dihydro-5,8-dihydroxy-9,10-dioxo-1,4-anthracenediyl)bis(imino-2,1-ethanediyl)bis[N-[4-[(2,5-dioxo-1-pyrrolidinyl)oxy]-1,4-dioxobutyl]-L-alpha-aspartyl-L-alanyl-L-leucinamide] bis(1,1-dimethylethyl)ester | |
| 113 | Ex. 94 | N,N'-[(9,10-Dihydro-5,8-dihydroxy-9,10-dioxo-1,4-anthracenediyl)bis(imino-2,1-ethanediyl)]bis[N-[1,4-dioxo-4-[2,5-dioxo-1-pyrrolidinyl)oxy]-1,4-dioxobutyl]-L-alpha-aspartylglycylglycyl-L-prolinamide] bis(1,1-dimethylethyl)ester | |
| 114 | Ex. 93 | N,N'-[(9,10-Dihydro-5,8-dihydroxy-9,10-dioxo-1,4-anthracenediyl)bis-(imino-2,1-ethanediyl)]bis[N-[4-[(2,5-dioxo-1-pyrrolidinyl)oxy]-1,4-dioxobutyl]glycylglycyl-L-alpha-aspartyl-L-alaninamide] bis(1,1-dimethylethyl)ester | |

Table V (continued)

| Example | Precursor | Compound | MP°C |
|---|---|---|---|
| 115 | Ex. 92 | N,N'-[(9,10-Dihydro-5,8-dihydroxy-9,10-dioxo-1,4-anthracenediyl)-bis(imino-2,1-ethanediyl)]bis[N-[4-[(2,5-dioxo-1-pyrrolidinyl)-oxy]-1,4-dioxobutyl]-L-alpha-aspartyl-L-seryl-L-alanyl-L-leucyl-L-leucinamide] bis(1,1-dimethylethyl)ester | |
| 116 | Ex. 88 | N,N'-[(9,10-Dihydro-5,8-dihydroxy-9,10-dioxo-1,4-anthracenediyl)-bis(imino-2,1-ethanediyl)]bis[N-[4-[(2,5-dioxo-1-pyrrolidinyl)-oxy]-1,4-dioxobutyl]-L-alpha-aspartyl-L-leucyl-L-alanyl-L-leucinamide] bis(1,1-dimethylethyl)ester | |
| 117 | Ex. 87 | N,N'-[(9,10-Dihydro-5,8-dihydroxy-9,10-dioxo-1,4-anthracenediyl)-bis(imino-2,1-ethanediyl)]bis[N-[4-[(2,5-dioxo-1-pyrrolidinyl)oxy]-1,4-dioxobutyl]glycylglycyl-L-phenylalanyl-L-leucinamide] | |
| 118 | Ex. 89 | N,N'-[(9,10-Dihydro-5,8-dihydroxy-9,10-dioxo-1,4-anthracenediyl)bis(imino-2,1-ethanediyl)]bis[N-[4-[(2,5-dioxo-1-pyrrolidinyl)oxy]-1,4-dioxobutyl]-L-leucyl-L-alanyl-L-leucyl-L-alpha-asparaginamide] bis(1,1-dimethylethyl)ester | |
| 119 | Ex. 90 | N,N'-[(9,10-Dihydro-5,8-dihydroxy-9,10-dioxo-1,4-anthracenediyl)-bis(imino-2,1-ethanediyl)]bis[N-[4-[(2,5-dioxo-1-pyrrolidinyl)-oxy]-1,4-dioxobutyl]glycylglycyl-L-alpha-aspartyl-L-alanyl-L-leucinamide] bis(1,1-dimethylethyl)ester | |

**Table V (continued)**

| Example | Precursor | Compound | MP°C |
|---|---|---|---|
| 120 | Ex. 91 | N,N'-[(9,10-Dihydro-5,8-dihydroxy-9,10-dioxo-1,4-anthracenediyl)-bis(imino-2,1-ethanediyl)]bis[N-[4-[(2,5-dioxo-1-pyrrolidinyl)-oxy]-1,4-dioxobutyl]glycyl-L-alpha-aspartyl-L-alanyl-L-leucinamide] bis(1,1-dimethylethyl)ester | |

## Synthesis of N-Hydroxysuccinimide Ester Compounds Via Deblocking of the Corresponding Aspartic (Tertiary-butyloxy) Compounds

### Example 121

**N,N'-[(9,10-Dihydro-5,8-dihydroxy-9,10-dioxo-1,4-anthracenediyl)bis(imino-2,1-ethanediyl)]-bis[N-[4-[(2,5-dioxo-1-pyrrolidinyl)oxy]-1,4-dioxobutyl]-L-α-aspartyl-L-alanyl-L-alaninamide]**

To a stirred slurry of 125 mg of N,N'-[(9,10-dihydro-5,8-dihydroxy-9,10-dioxo-1,4-anthracenediyl)bis-(imino-2,1-ethanediyl)]bis[N-[4-[(2,5-dioxo-1-pyrrolidinyl)oxy]-1,4-dioxobutyl]-L-α-aspartyl-L-alanyl-L-alaninamide] bis(1,1-dimethylethyl)ester in 1.25 ml of anisole, cooled in an ice bath, was added 2.5 ml of trifluoroacetic acid. After 15 minutes the ice bath was removed and the reaction mixture was stirred for 75 additional minutes. Then the mixture was poured into diethyl ether and the solid was collected by filtration and dried to yield 98 mg of the desired product in 86% yield.

Additional N-hydroxysuccinimide ester compounds which were synthesized via deblocking of the corresponding aspartic (tertiary-butyloxy) compounds in a similar manner to that described in Example 121 are listed in Table VI as Examples 122-129.

## Table VI

**N-HYDROXYSUCCINIAMIDE ESTER SYNTHESIS VIA DEBLOCKING OF THE CORRESPONDING ASPARTIC (TERTIARY-BUTYLOXY) COMPOUNDS**

| Example | Precursor | Compound |
| --- | --- | --- |
| 122 | Ex. 112 | N,N'-[(9,10-Dihydro-5,8-dihydroxy-9,10-dioxo-1,4-anthracenediyl)-bis(imino-2,1-ethanediyl)]bis[N-[4-[(2,5-dioxo-1-pyrrolidinyl)-oxy]-1,4-dioxobutyl]-L-alpha-aspartyl-L-alanyl-L-leucinamide] |
| 123 | Ex. 115 | N,N'-[(9,10-Dihydro-5,8-dihydroxy-9,10-dioxo-1,4-anthracendiyl)-bis(imino-2,1-ethanediyl)]bis[N-[4-[(2,5-dioxo-1-pyrrolidinyl)-oxy]-1,4-dioxobutyl]-L-alpha-aspartyl-L-seryl-L-alanyl-L-leucyl-L-leucinamide] |
| 124 | Ex. 114 | N,N'-[(9,10-Dihydro-5,8-dihydroxy-9,10-dioxo-1,4-anthracenediyl)-bis(imino-2,1-ethanediyl]bis[N-[4-[(2,5-dioxo-1-pyrrolidinyl)oxy]-1,4-dioxobutyl]glycylglycyl-L-alpha-aspartyl-L-alaninamide] |
| 125 | Ex. 113 | N,N'-[(9,10-Dihydro-5,8-dihydroxy-9,10-dioxo-1,4-anthracenediyl)bis(imino-2,1-ethanediyl)]bis[N-[4-[(2,5-dioxo-1-pyrrolidinyl)oxy]-1,4-dioxobutyl]-L-alpha-aspartylglycyl-L-alanyl-L-prolinamide] |

**Table VI (continued)**

| Example | Precursor | Compound | % yield |
|---|---|---|---|
| 126 | Ex. 116 | N,N'-[(9,10-Dihydro-5,8-dihydroxy-9,10-dioxo-1,4-anthracenediyl)-bis(imino-2,1-ethanediyl)]bis[N-[4-(2,5-dioxo-1-pyrrolidinyl)oxy]-1,4-dioxobutyl]-L-alpha-aspartyl-L-leucyl-L-alanyl-L-leucinamide] | |
| 127 | Ex. 118 | N,N'-[(9,10-Dihydro-5,8-dihydroxy-9,10-dioxo-1,4-anthracenediyl)-bis(imino-2,1-ethanediyl)]bis[N-[4-[(2,5-dioxo-1-pyrrolidinyl)-oxy]-1,4-dioxobutyl]-L-leucyl-L-alanyl-L-leucyl-L-alpha-asparagin-amide] | |
| 128 | Ex. 119 | N,N'-[(9,10-Dihydro-5,8-dihydroxy-9,10-dioxo-1,4-anthracenediyl)-bis(imino-2,1-ethanediyl)]bis[N-[4-[(2,5-dioxo-1-pyrrolidinyl)-oxy]-1,4-dioxobutyl]glycylglycyl-L-alpha-aspartyl-L-alanyl-L-leucinamide] | |
| 129 | Ex. 120 | N,N'-[(9,10-Dihydro-5,8-dihydroxy-9,10-dioxo-1,4-anthracenediyl)-bis(imino-2,1-ethanediyl)] bis[N-[4-(2,5-dioxo-1-pyrrolidinyl)oxy]-1,4-dioxobutyl]glycyl-L-alpha-aspartyl-L-alanyl-L-leucinamide] | |

Example 130

## Preparation of a Monoclonal Antibody T-101 Conjugate with N,N'-[(9,10-Dihydro-5,8-dihydroxy-9,10-dioxo-1,4-anthracenediyl)-bis(imino-2,1-ethanediyl)]-bis[N-[4-[(2,5-dioxo-1-pyrrolidinyl)oxy]-1,4-dioxobutyl]-L-α-aspartyl-L-seryl-L-alanyl-L-leucyl-L-leucinamide]

A 38 fold molar excess (2.0 mg) of the N-hydroxysuccinimide ester N,N'-[(9,10-dihydro-5,8-dihydroxy-9,10-dioxo-1,4-anthracenediyl)bis(imino-2,1-ethanediyl)bis[N-[4-[(2,5-dioxo-1-pyrrolidinyl)oxy]-1,4-dioxobutyl]-L-α-aspartyl-L-seryl-L-alanyl-L-leucyl-L-leucinamide], also referred to as "drug", in 0.2 ml of N,N-dimethylformamide was added to 5.0 mg of T-101 antibody in 3.0 ml of 50mM pH 8 sodium phosphate buffer. The sample was capped and incubated in a 37°C water bath, with mild agitation for 18 hours. Then a 50 fold molar excess of 0.1M glycine (0.25 ml) in pH 8 phosphate buffer was added, incubating the mixture at 30°C for 2 hours. An 80% v/v reduction was performed by sealing the reaction mixture in a 50,000d molecular weight cut-off dialysis bag surrounded by Aquacide III. The concentrated mixture was then applied to a 1 x 28 cm Sephadex G-25 column, equilibrated and eluted with 50mM pH 7 sodium phosphate buffer. The eluate was concentrated to 1-1.5 ml with Aquacide III, then was exhaustively dialyzed (using three-two liter portions of 50mM sodium phosphate/150mM sodium chloride/pH 7, then two liters of 50 mM sodium phosphate pH 7 buffer alone) at 4°C. The sample volume was adjusted to 1.3-1.5 ml and the solution was filter sterilized (Millipore® or Corning® 0.45 millimicron discs). The final product was analyzed for drug and protein content by size exclusion high pressure liquid chromatography ("SEC HPLC"), ultraviolet ("uv") spectroscopy and visible spectroscopy. SEC HPLC was performed using a BIORAD® TSK 250 column equilibrated with 100 mM, pH 7 sodium phosphate buffer.

An HPLC retention time profile curve with either single (280nm) or multiple (280, 616 or 668nm) uv/visible detection was generated. By using the extinction coefficients of the antibody and drug (actually the succinic acid corresponding to the N-hydroxysuccinimide ester used in conjugation) at these wavelengths, the number of drug molecules per molecule of antibody, and the concentration of the conjugate was calculated.

If desired, due to the individual characteristics of the various antibodies and drug N-hydroxysuccinimide esters, improvements in the results of a given conjugation experiment can be effected by manipulation of a variety of parameters. These include time, temperature, concentration, mole ratio, speed of addition of the drug ester, presence of solubilizing agents such as glycerol, and pH.

Further purification of conjugates by gel or HPLC may also be performed.

Molar Drug Loadings of Drug-Antibody Conjugates

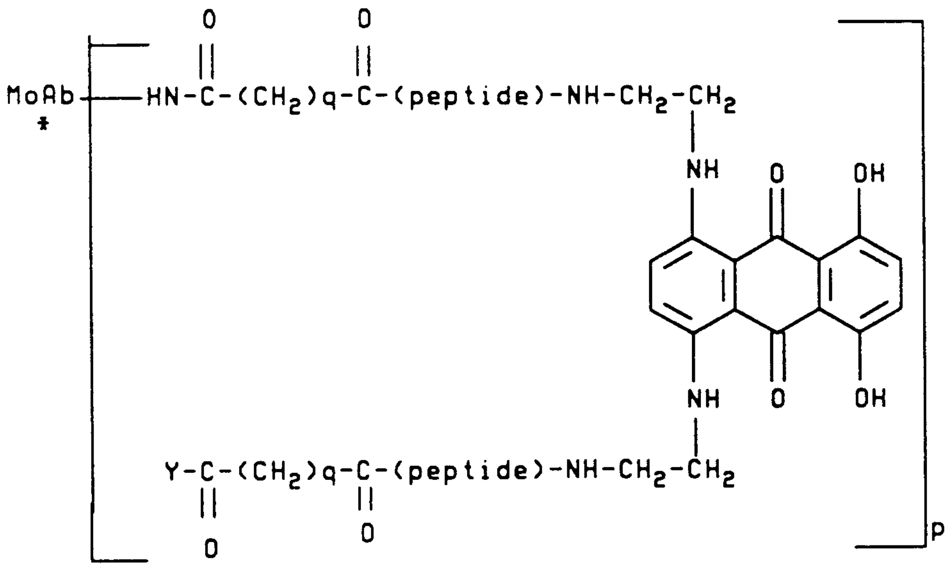

**Y = OH or a bond to the antibody**

**q = 1 to 4**

Table VII shows the values of p in the above formula as determined for drug-antibody conjugates of the listed peptides and monoclonal antibodies.

## Table VII

| Peptide | of Example no. | Loading (p) Values for Given Monoclonal Antibodies (MoAb) | | | | |
|---|---|---|---|---|---|---|
| | | MX-1/16.4 | MX-1/57/1.4 | MX-1/6.2 | T-101 | P96.5 |
| -Asp-Ala-Leu- | 98 | 8 | | | 7 | 6 |
| -Asp-Ala-Ala- | 97 | 15 | | | | |
| -Asp(O-t-Bu)-Ala-Ala- | 70 | 2.6 | | | | |
| -Asp-Ser-Ala-Leu-Leu- | 102 | | 9 | 4 | 11 | 14 |
| -Asp-Leu-Ala-Leu- | 99 | | | | 3.5 | 6.6 |
| -Gly-Phe-Phe-Leu- | 87 | | 24 | | 0 | |
| -Gly-Gly-Asp-Ala- | 103 | 1.2 | | 2.4 | 2.8 | 4.3 |

**Table VII (continued)**

| Peptide | of Example no. | Loading (p) Values for Given Monoclonal Antibodies (MoAb) MX-1/16.4 | MX-1/57/1.4 | MX-1/6.2 | T-101 | P96.5 |
|---|---|---|---|---|---|---|
| -Leu-Ala-Leu-Asp | 100 | | | | 2.1 | |
| -Gly-Gly-Asp-Ala-Leu- | 101 | | | | 3.5 | 4.6 |
| -Asp-Gly-Ala-Pro- | 125 | | | | 6.3 | |
| -Gly-Asp-Ala-Leu | 95 | | | | 2.1 | 3.5 |

## Claims

**1.** Compounds of the formula:

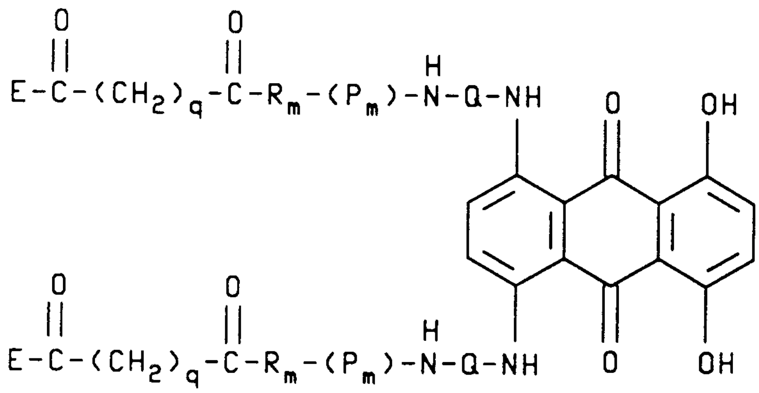

wherein Q is a divalent moiety selected from the group consisting essentially of

$-(CH_2)_n-$, 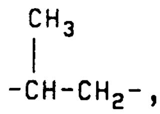, 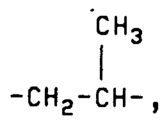, 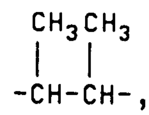,

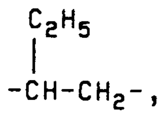, 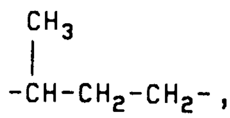, 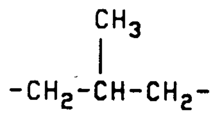

and 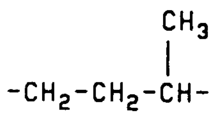

where n is an integer from 2 to 4 inclusive; R is selected from the group of D or L amino acids consisting essentially of cysteine, leucine, isoleucine, phenylalanine, tyrosine, proline, tryptophan, hydroxyproline, aspartic acid, asparagine, glutamic acid, glutamine, lysine, ornithine, arginine, histidine, alanine, glycine, methionine, valine, threonine and serine which are linked through amide bonds between the $\alpha$-amino functionality of one amino acid and the carboxyl group of the adjacent amino acid, and in those amino acids which possess side-chain functionality, the side-chains can be optionally substituted by alkyl or aryl amino acid side-chain protecting groups (P); E is hydroxyl or an ester; m is an integer from 1 to 10, inclusive; q is an integer from 1 to 4, inclusive; and the pharmacologically acceptable organic or inorganic acid addition salts or combination of salts thereof.

**2.** Compounds in accordance with Claim 1, wherein q is 2; Q is $-CH_2-CH_2-$; R is an L amino acid; (P) is a tertiary-butyl ester; E is hydroxyl or an N-hydroxysuccinimide ester; m is an integer from 2 to 6 inclusive and the compound is a hydrogen bromide or hydrogen chloride salt.

**3.** A method of treating tumors in warm-blooded animals which comprises administering to said animals an effective amount of a compound of Claim 1.

**4.** A composition of matter in dosage unit form comprising from about 1 $mg/m^2$/day to about 1.2 $g/m^2$/day of mammalian body surface area of a compound of Claim 1 in association with a pharmacologically acceptable carrier.

**5.** A conjugate of the formula:

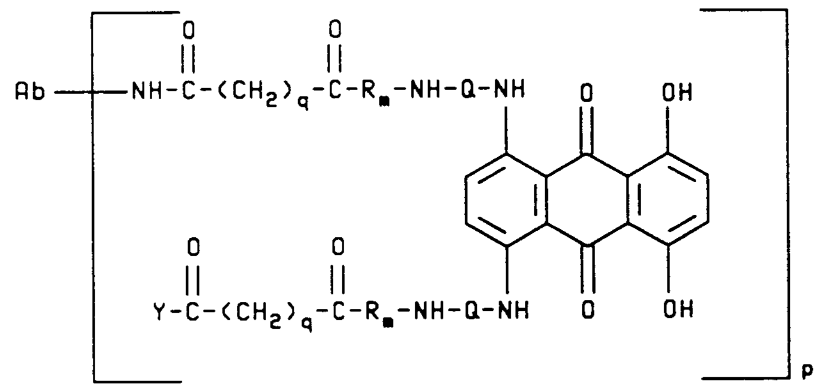

where Q is a divalent moiety selected from the group consisting essentially of

$-(CH_2)_n-$, 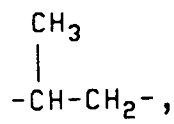, 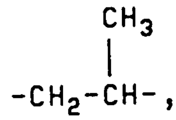, 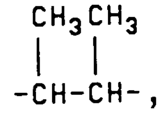,

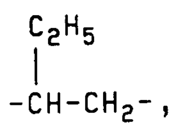, 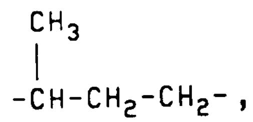, 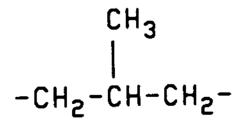

and 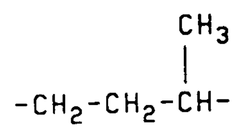

where n is an integer from 2 to 4 inclusive; $R_m$ is selected from the group of D or L amino acids consisting essentially of cysteine, leucine, isoleucine, phenylalanine, tyrosine, proline, tryptophan, hydroxyproline, aspartic acid, asparagine, glutamic acid, glutamine, lysine, ornithine, arginine, histidine, alanine, glycine, methionine, valine, threonine and serine which are linked through amide bonds between the α-amino functionality of one amino acid, and the carboxyl group of the adjacent amino acid, and m is an integer from 1 to 10, inclusive; q is an integer from 1 to 4, inclusive; Ab is a monoclonal or polyclonal antibody or fragments thereof; Y is hydroxyl or a bond to the antibody; p is the number of molecules of drug covalently attached; and the pharmacologically acceptable acid addition salts thereof.

6. A conjugate in accordance with Claim 5, wherein Q is $-CH_2-CH_2-$, at least one $R_m$ is aspartic or glutamic acid, Ab is a monoclonal antibody recognizing a human tumor and p is an integer from 2 to 20.

7. A method of targeting and treating tumors in warm-blooded animals which comprises administering to said animals an effective amount of a conjugate of Claim 5.

8. A composition of matter in dosage unit form comprising from about 3 $mg/m^2$/day to about 2 $g/m^2$/day of mammalian body surface area of a conjugate of Claim 5 in association with a pharmaceutically acceptable carrier.

9. A process for preparing compounds of Claim 1 comprising (a):

reacting a compound of formula:

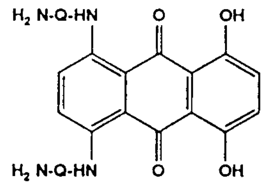

wherein Q is a divalent moiety as hereinabove defined, by dissolving the compound in a chilled solvent such as N,N-dimethylformamide containing an organic base such as triethylamine or diisopropylethylamine, or alternatively, in dry tetrahydrofuran in the presence of trimethylsilyl chloride and triethylamine, with an amino acid of formula:

$L_1R_1(P_1)A_1$

wherein $R_1$ is an L- or D-amino acid core, $L_1$ is an amine protecting group selected from tertiary-butyloxycarbonyl, benzyloxycarbonyl, fluorenylmethoxycarbonyl, and the like, $A_1$ is a group introduced to activate the amino acid carboxyl group toward nucleophylic substitution and is defined, together with $R_1$, as a hydroxysuccinimide ester or a symmetrical or an unsymmetrical anhydride, and $P_1$ is a side-chain amino acid protecting group used for those amino acids which require protection during peptide assembly, and is defined as, for example, tertiary-butyloxy or benzyloxy when $R_1$ is derived from aspartic or glutamic acid; carbobenzyloxycarbonyl or tertiary-butyloxycarbonyl when $R_1$ is derived from lysine or ornithine or arginine; or tertiarylbutyl, tertiary-butylthio, or acetyl when $R_1$ is derived from cysteine; dropwise adding $L_1R_1(P_1)A_1$ dissolved into the same solvent system described for the above anthraquinone to the solution of the anthraquinone, stirring the solution at a temperature of between 0°C to 400°C for about 24 hours, filtering the reaction, and evaporating the solution or adding a solvent of low polarity to precipitate a compound of formula:

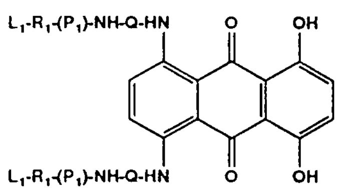

wherein $L_1$, $R_1$, and $P_1$ and Q are as hereinabove defined; removing the $\alpha$-amine protecting group $L_1$ to produce a compound of formula:

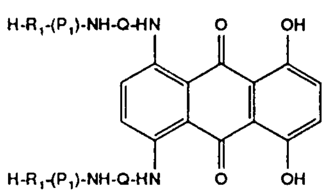

treating the immediately hereinabove compound with a compound of formula:

$L_2R_2(P_2)A_2$

where $L_2$, $R_2$, $P_2$, and $A_2$ are as hereinbefore defined for $L_1$, $R_1$, $P_1$, and $A_1$ and using the conditions as hereinbefore defined for the use of $L_1R_1(P_1)A_1$ to produce, after cleavage of the group $L_2$, a compound of the formula:

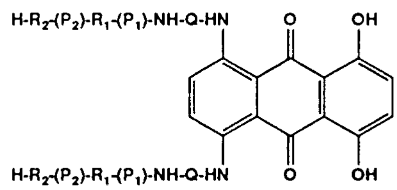

then treating the immediately hereinabove compound in sequence with additional compounds of the general formula $L_nR_n(P_n)A_n$ wherein $L_n$, $R_n$, $P_n$, and $A_n$ are as hereinbefore defined for $L_1$, $R_1$, $P_1$, $A_1$, and n is defined as an integer from 0 to 8, inclusive, using the conditions as hereinbefore defined for the use of $L_1R_1(P_1)A_1$ as many times as appropriate to produce, after cleavage of the group $L_2$, a compound of the formula:

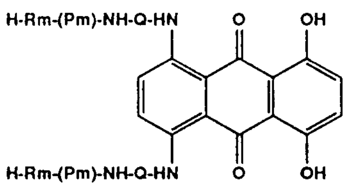

or (b):

reacting a compound of the formula:

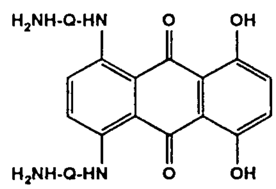

with a preassembled protected, activated peptide of formula:

$L_mR_m(P_m)A_m$

wherein $L_m$, $R_m$, $P_m$, and $A_m$ are as hereinbefore defined for $L_1$, $R_1$, $P_1$, $A_1$, and m is defined as an integer from 1 to 10, inclusive, using the conditions as hereinbefore defined for the use of $L_1R_1(P_1)A_1$ to produce, after cleavage of the group $L_m$, a compound of the formula:

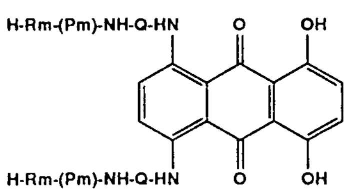

then dissolving the compound prepared as described under (a) or (b) above into a solvent such as N,N-dimethylformamide in the presence of a tertiary amine base such as triethylamine, diisopropylethylamine of N-methylmorpholine with a compound of the formula:

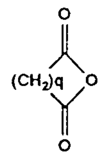

wherein m is an integer from 1 to 4, inclusive, with stirring, at room temperature for from 8 to 48 hours, diluting the reaction solution with a solvent such as diethyl ether, filtering the precipitate, and triturating this with dilute phosphoric acid to produce a compound of the formula:

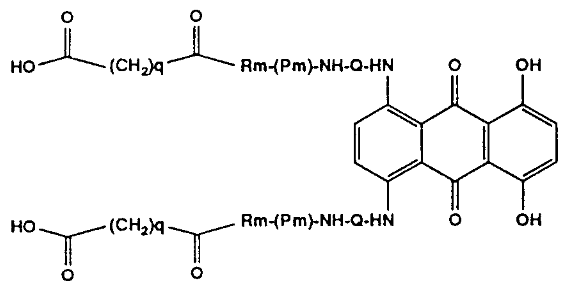

wherein $R_m$, $P_m$, Q, and q are as hereinbefore defined, then the above product is dissolved in a solvent such as N,N-dimethylformamide and treated with about 2.2 equivalents of N-hydroxysuccinimide and about 2.2 equivalents of dicyclohexylcarbodiimide, then stirred at about room temperature for from 16 to 74 hours, if necessary making several more additions of 1.1 equivalents in portions of N-hydroxysuccinimide and dicyclohexylcarbodiimide until the absence of the above material is evidenced by an analytical technique such as thin layer chromatography, then filtering the solution, evaporating the solvents, triturating the residue with a solvent such as ethyl acetate, filtering, and purifying by stirring the residue with a solvent such as isopropyl alcohol to produce the hydroxysuccinimide ester product of the formula:

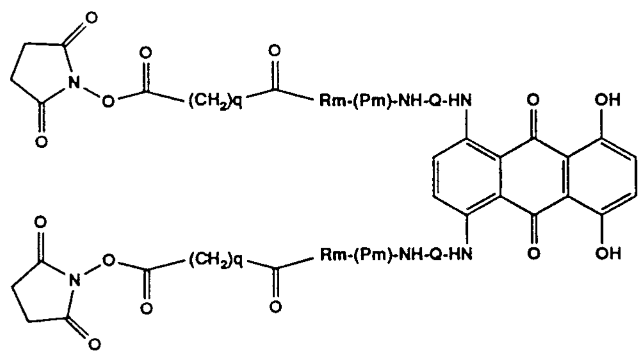

then removing the sidechain protecting groups $P_m$: if one or more of the groups $P_m$ is tertiary-butyloxy or tertiary-butyloxycarbonyl, this is done by dissolving the above compound in a mixture of acetic acid and anisole, bubbling hydrogen chloride gas into the solution for a few minutes, allowing the solution to stand at room temperature for from one to 24 hours, then isolating the product by precipitation with a solvent of low polarity; or if one or more of the groups $P_m$ is benzyloxycarbonyl, it is removed by dissolving the above product in a solvent such as acetic acid, cooling, bubbling gaseous hydrobromic acid through the solution, leaving the reaction solution at room temperature for one to 24 hours, and isolating product by precipitation; removal of all the protecting groups $P_m$ produces a compound of the formula:

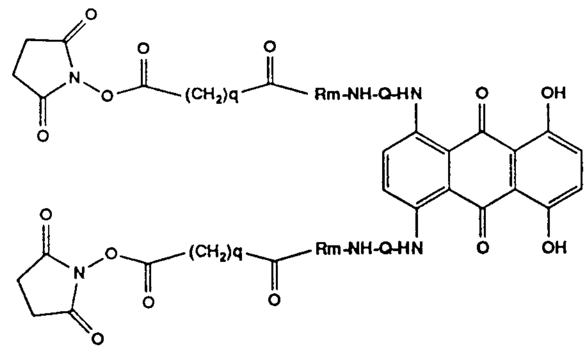

wherein $R_m$, Q, Y and q are as hereinabove defined.

**10.** A process for preparing a conjugate of Claim 5 comprising dissolving a 10 to 100-fold molar excess of a compound of the formula:

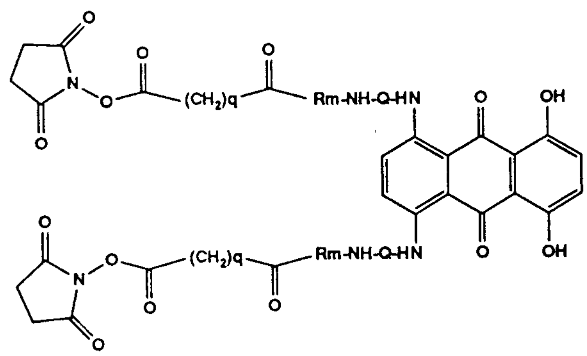

wherein $R_m$, Q, and q are as hereinabove defined, into the minimal amount of a polar solvent such as N,N-dimethylformamide that affects dissolution, then adding this solution to a solution of antibody in a basic buffer such as 50mM pH 8 sodium phosphate buffer, the second solution containing from one to 10 mg of antibody per milliliter of buffer, then incubating the mixture at from room temperature to about 40° for from four to 24 hours; after the specified amount of time, a solution of 20 to 200-fold molar excess of an amino acid such as glycine is added, and incubation of the solution is continued for about 2 hours; a reduction in solution volume is then done by using a technique such as ultrafiltration or dialysis, the volume-reduced solution is applied to a size-exclusion column such as Sephadex G-25, the column is eluted with a neutral buffer such as 50mM pH 7 sodium phosphate, the appropriate eluate fractions are dialyzed and filter sterilized to product an antibody conjugate of the formula:

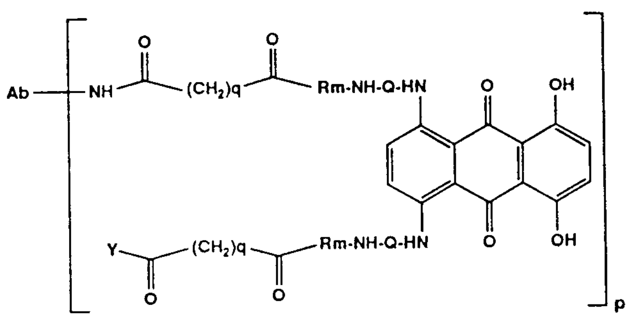

where Ab, q, $R_m$, Q, Y and p are as hereinabove defined.

European Patent Office

**PARTIAL EUROPEAN SEARCH REPORT**
which under Rule 45 of the European Patent Convention shall be considered, for the purposes of subsequent proceedings, as the European search report

Application number
EP 91100267.3

## DOCUMENTS CONSIDERED TO BE RELEVANT

| Category | Citation of document with indication, where appropriate, of relevant passages | Relevant to claim | CLASSIFICATION OF THE APPLICATION (Int Cl.⁵) |
|---|---|---|---|
| D,A | G. GREGORIADIS et al. "Targeting of Drugs", 1982, PLENUM PRESS, New York, A. TROUET et al. "Targeting of Antitumour and Antiprotozoal drugs by covalent linkage to protein carries * pages 19-30 * Totality * | 1,9 | A 61 K 47/48<br>C 07 K 5/00<br>C 07 K 7/00<br>A 61 K 37/02<br>A 61 K 39/395<br>A 61 K 31/12 |
| A | CHEMICAL ABSTRACTS, vol. 100, no. 11, March 12, 1984, Columbus, Ohio, USA S. E. LOCHER et al. "Relationship between cytotoxicity and DNA damage in Mammalian cells treated with anthracenedione derivatives." pages 23,24, abstract-no. 79 558c & Chem.-Biol. Interact. 1983, 46(3), 369-79 | 1,4 | |
| | | | TECHNICAL FIELDS SEARCHED (Int Cl.⁵) |
| A | DE - A - 3 907 290 | 1 | A 61 K<br>C 07 K |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1,2,4-6,8-10
Claims searched incompletely: -
Claims not searched: 3,7
Reason for the limitation of the search:

Article 52(4) EPC

| Place of search | Date of completion of the search | Examiner |
|---|---|---|
| VIENNA | 13-03-1992 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 03.82

European Patent Office

# PARTIAL EUROPEAN SEARCH REPORT

Application number
-2-
EP 91100267.3

| DOCUMENTS CONSIDERED TO BE RELEVANT | | | CLASSIFICATION OF THE APPLICATION (Int. Cl. 5) |
|---|---|---|---|
| Category | Citation of document with indication, where appropriate, of relevant passages | Relevant to claim | |
| | (EISENBRAND) * Totality, especially page 2, lines 23,24,61-64, page 3, lines 23-60 * ---- | | |
| | | | TECHNICAL FIELDS SEARCHED (Int. Cl.4) |